# EUROPÄISCHE PATENTSCHRIFT

(11) **EP 1 353 927 B1**
(45) Veröffentlichungstag und Bekanntmachung des Hinweises auf die Patenterteilung: **24.08.2005**
(21) Anmeldenummer: 01271992.8
(22) Anmeldetag: 03.12.2001
(51) Int. Cl.: C07D 495/00

(54) **SULFAMIDOTHIENOPYRIMIDINE ZUR VERWENDUNG ALS PHOSPHODIESTERASE V-HEMMER**
SULFAMIDOTHIENOPYRIMIDINES AND THE USE OF THE SAME AS PHOSPHODIESTERASE V INHIBITORS
SULFAMIDOTHIENOPYRIMIDINES ET LEUR UTILISATION COMME INHIBITEURS DE PHOSPHODIESTERASE V

(30) Priorität: 23.12.2000 DE 10064994
(43) Veröffentlichungstag der Anmeldung: 22.10.2003
(73) Patentinhaber: Merck Patent GmbH, 64293 Darmstadt (DE)
(72) Erfinder: EGGENWEILER, Hans-Michael, 64287 Darmstadt (DE); SCHWARTZ, Harry, 65719 Hofheim (DE); SCHELLING, Pierre, 64367 Mühltal (DE); BEIER, Norbert, 64354 Reinheim (DE); CHRISTADLER, Maria, 63322 Rödermark (DE)
(86) Internationale Anmeldenummer: PCT/EP2001/014102
(87) Internationale Veröffentlichungsnummer: WO 2002/051848

(56) Entgegenhaltungen:
- WO-A-00/59912

## Beschreibung

Die Erfindung betrifft Verbindungen der Formel I 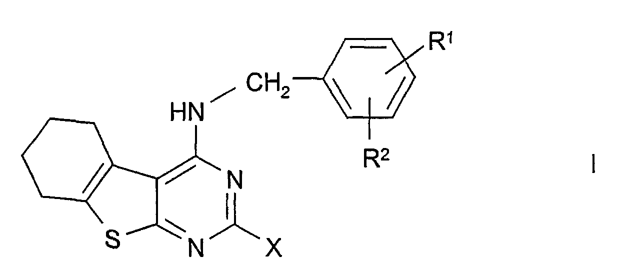 worin
- R¹, R²: jeweils unabhängig voneinander H, OA¹, OCOA¹, OH oder Hal, wobei R¹ und R² zusammen auch Alkylen mit 3-5 C-Atomen, -O-CH₂-CH₂-, -CH₂-O-CH₂-, -O-CH₂-O- oder -O-CH₂-CH₂-O-,
- X: -CH₂SO₂NR³R⁴,
- R³, R⁴: unabhängig voneinander H, ein Heteroaromat, unsubstituiertes oder terminal durch -NH₂, NHA¹ oder -NA¹A² substituiertes Alkyl oder Alkenyl mit 1-10 C-Atomen, worin eine oder zwei CH₂-Gruppen durch -CH=CH-Gruppen, -O-, -NH- oder -NA¹- ersetzt sein können, wobei R³ und R⁴ zusammen auch unsubstituiertes oder ein- oder mehrfach durch -Hal, -NH₂, -NHA, -NA¹A², -NHCOA¹, NHCOOA¹, -COOH, -COOA¹, -CONH₂, -CONHA¹, oder -CONA¹A² substituiertes Cycloalkyl oder Cycloalkylen mit 3-7 C-Atomen, worin eine oder zwei-CH₂-Gruppen durch -O-, -NH-, -NA¹-, -NCOA¹- oder -NCOOA¹ ersetzt sein können
- A¹, A²: jeweils unabhängig voneinander Alkyl oder Alkenyl mit 1 bis 10 C-Atomen, das durch 1 bis 5 F- und/oder Cl-Atome substiutiert sein kann, wobei A¹ und A² zusammen auch Cycloalkyl oder Cylcloalkylen mit 3-7 C-Atomen, worin eine CH₂Gruppe durch -O-, -NH-, -NA¹-, NCOA¹- oder -NCOOA¹- ersetzt sein kann,
und
- Hal: F, Cl, Br oder I
bedeuten,
sowie deren Salze und/oder Solvate.

Pyrimidinderivate sind beispielsweise aus der DE 19819023, EP 201 188 oder der WO 93/06104 bekannt.
Der Erfindung lag die Aufgabe zugrunde, neue Verbindungen mit wertvollen Eigenschaften aufzufinden, insbesondere solche, die zur Herstellung von Arzneimitteln verwendet werden können.
Es wurde gefunden, daß die Verbindungen der Formel I und ihre Salze bei guter Verträglichkeit sehr wertvolle pharmakologische Eigenschaften besitzen.
Insbesondere zeigen sie eine spezifische Inhibierung der cGMP-Phosphodiesterase (PDE V).
Im Vergleich zu den Verbindungen aus dem Stand der Technik, weisen die erfindungsgemäßen Verbindungen günstigere physikalisch-chemische Eigenschaften auf. So sind sie besser löslich und werden z.B. bei oraler Gabe besser resorbiert.
Chinazoline mit cGMP-Phosphodiesterase hemmender Aktivität sind z.B. in J. Med. Chem. 36, 3765 (1993) und ibid. 37, 2106 (1994) beschrieben.

In WO 00/59912 werden tricyclische Thieno(2,3-d)pyrimidine beschrieben, die einen cGMP-spezifischen Phosphordiesterase hemmenden Effekt aufweisen, sich aber strukturell von den Verbindungen der vorliegenden Anmeldung unterscheiden.

Die biologische Aktivität der Verbindungen der Formel I kann nach Methoden bestimmt werden, wie sie z.B in der WO 93/06104 beschrieben sind. Die Affinität der erfindungsgemäßen Verbindungen für cGMP- und cAMP-Phosphodiesterase wird durch die Ermittlung ihrer IC₅₀-Werte (Konzentration des Inhibitors, die benötigt wird, um eine 50 %ige Inhibierung der Enzymaktivität zu erreichen) bestimmt.
Zur Durchführung der Bestimmungen können nach bekannten Methoden isolierte Enzyme verwendet werden (z.B. W.J. Thompson et al., Biochem. 1971, 10, 311). Zur Durchführung der Versuche kann eine modifizierte "batch"-Methode von W.J. Thompson und M.M. Appleman (Biochem. 1979, 18, 5228) angewendet werden.

Die Verbindungen eignen sich daher zur Behandlung von Erkrankungen des Herz-Kreislaufsystems, insbesondere der Herzinsuffizienz und zur Behandlung und/oder Therapie von Potenzstörungen (erektile Dysfunktion).

Die Verwendung von substituierten Pyrazolopyrimidinonen zur Behandlung von Impotenz ist z.B. in der WO 94/28902 beschrieben.

Die Verwendung anderer PDE V-Hemmer ist beschrieben z.B. in der WO 94/28902.

Die Verbindungen sind wirksam als Inhibitoren der Phenylephrin-induzierten Kontraktionen in Corpus cavernosum-Präparationen von Hasen.
Diese biologische Wirkung kann z.B. nach der Methode nachgewiesen werden, die von F. Holmquist et al. in J. Urol., 150, 1310-1315 (1993) beschrieben wird.
Die Inhibierung der Kontraktion, zeigt die Wirksamkeit der erfindungsgemäßen Verbindungen zur Therapie und/oder Behandlung von Potenzstörungen.

Pharmazeutische Formulierungen bestehend aus anderen Phosphodiesterase V (PDE V)-Hemmern zusammen mit einem zweiten Wirkstoff sind in der WO 00/15639 beschrieben.
Andere Kombinationen kennt man aus der WO 00/15228.

Pharmazeutische Formulierungen bestehend aus anderen Phosphodiesterase V (PDE V)-Hemmern zusammen mit einem Prostaglandin oder Prostaglandinderivat sind in der WO 00/15639 und WO 0015228 beschrieben.

Die Verwendung von (anderen) Phosphodiesterase IV oder V Hemmern in Kombination mit einem Prostaglandin oder Prostaglandinderivat zur lokalen Behandlung von erektiler Dysfunktion ist in der WO 9921558 beschrieben.

R.T. Schermuly et al. beschreiben im *American Journal of Respiratory and Critical Care Medicine,* 160, 1500-6 (1999), die therapeutische Möglichkeit Prostaglandin I₂ (PGI₂) in Aerosolform mit systemischen PDE Inhibitoren, vorzugsweise dual-selektiven PDE III/IV Inhibitoren, in niedriger Dosierung bei akutem und chronischen pulmonalem Hochdruck zu verwenden.

In *Pneumologie* (54, Suppl. 1, S42, 2000) wird von R. Schermuly et al. der Einfluß der PDE V-Inhibierung auf die durch Prostacyclin induzierte Vasorelaxation bei experimenteller pulmonaler Hypertonie beschrieben.

Pharmazeutische Formulierungen bestehend aus anderen Phosphodiesterase V (PDE V)-Hemmern zusammen mit Calcium-Antagonisten (= Calciumkanalblocker) sind in der WO 00/15639 beschrieben.

Kombinationen von PDE V - Hemmern mit Endothelin-Rezeptor-Antagonisten sind z.B in der WO 99/64004 beschrieben.

Pharmazeutische Formulierungen bestehend aus anderen Phosphodiesterase V (PDE V)-Hemmern zusammen mit einem Nitrat sind in der WO 00/15228 beschrieben.
Die bekannte Kontraindikation der Gabe von Nitraten bei gleichzeitiger Einnahme von PDE V - Hemmern bei der Indikation erektile Dysfunktion ist z.B. in der WO 00/10542 beschrieben. Gleichzeitig wird dort jedoch offenbart, daß Nitrate als antianginöse Mittel verabreicht werden können, obwohl gleichzeitig Phosphodiesterase V - Hemmer zur Behandlung erektiler Dysfunktion eingesetzt werden.
Weiter werden dort pharmazeutische Zubereitungen beschrieben, die sowohl ein Nitrat als auch einen Phosphodiesterasehemmer enthalten, zur Anwendung in der Therapie erektiler Dysfunktion und/oder in der Therapie von Herz- /Kreislauferkrankungen bei gleichzeitigem Vorliegen der jeweils anderen Indikation.

Die erfindungsgemäßen Verbindungen eignen sich weiterhin zur Behandlung von Angina, Bluthochdruck, pulmonalem Hochdruck, congestivem Herzversagen, Herzinfarkt, chronischer obstruktiver pulmonaler Krankheit (COPD), Cor pulmonale, Rechtsherzinsuffizienz, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose, zur Behandlung weiblicher Sexualstörungen, Entzündungen, Osteoporose, weiterhin zur Behandlung von maligner Hypertonie, Phäochromazytom (katecholaminproduzierender Tumor der Nebennierenrinde), bei peripheren Gefäß(-verschluß)-erkrankungen, Gefäßerkrankungen, Thrombozytopenie, Ulcus pepticum (gutartiges Darmgeschwür), Darmbewegungsstörungen, perkutaner transluminaler Koronarangioplastie, Karotis Angioplastie, postoperativer Stenose des Koronarbypasses, Vorwehen sowie benigner Prostata-Hyperplasie.

Gegenstand der Erfindung sind somit die Verbindungen der Formel I sowie deren Salze und Solvate, inbesondere deren physiologisch unbedenklichen Salze und/oder Solvate.

Die Verbindungen der Formel I können als Arzneimittelwirkstoffe in der Human- und Veterinärmedizin eingesetzt werden. Ferner können sie als Zwischenprodukte zur Herstellung weiterer Arzneimittelwirkstoffe eingesetzt werden.

Vor- und nachstehend haben die Reste R¹, R², R³, R⁴, A¹, A², X und L die bei den Formeln I, II und III angegebenen Bedeutungen, sofern nicht ausdrücklich etwas anderes angegeben ist.

R¹ und R² bedeuten vorzugsweise unabhängig voneinander H, OA¹ oder Hal, insbesondere Alkoxy mit 1 bis 9 C-Atomen, Cl, Br oder F.

R³ und R⁴ bedeuten unabhängig voneinander bevorzugt H, einen Heteroaromaten oder terminal durch -NH₂, -NHA¹ oder -NA¹A² substituiertes Alkyl mit 1 bis 10 C-Atomen, worin A¹ die oben angegebene Bedeutung aufweist. Ferner bedeuten R³ und R⁴ zusammen bevorzugt auch unsubstituiertes oder ein- oder mehrfach durch -NH₂, -NHA, -NA¹A², -NHCOA¹, NHCOOA¹, -COOH, -COOA¹, -CONH₂, -CONHA¹, oder -CONA¹A² substituiertes Cycloalkyl oder Cycloalkylen mit 3-7 C-Atomen, worin eine oder zwei -CH₂-Gruppen durch -O-, -NH-, -NA¹-, -NCOA¹- oder -NCOOA¹ ersetzt sein können. Insbesondere bedeutet R³ und R⁴ unabhängig voneinander H, terminal durch -NH₂, -NHCH₃, -N(CH₃)₂ substituiertes Alkyl mit 1 bis 8 C-Atomen oder eine der folgenden Gruppen: 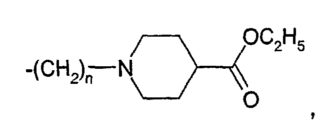 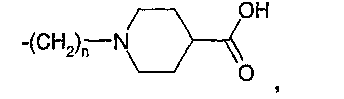 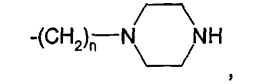 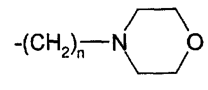 oder 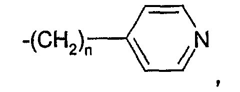 worin n 0, 1, 2, 3, 4, 5, 6, 7, 8, 9 oder 10, vorzugsweise 0 oder 2 bedeutet.

In den vorstehenden und nachfolgenden Formeln ist Alkyl vorzugsweise unverzweigt und hat 1, 2, 3, 4, 5, 6, 7 oder 8 C-Atome und bedeutet insesondere bevorzugt Methyl, Ethyl oder Propyl, weiterhin Isopropyl, Butyl, Isobutyl, sek.-Butyl oder tert.-Butyl, aber auch n-Pentyl, Neopentyl, lsopentyl oder Hexyl.

A¹ und A² bedeuten jeweils unabhängig voneinander bevorzugt Alkyl mit 1 bis 7 C-Atomen, wobei A¹ und A² zusammen auch Cycloalkylen mit 3-7 C-Atomen, worin eine CH₂-Gruppe durch -O-, -NH-, -NA¹-, -NCOA¹- oder-NCOOA¹- ersetzt sein kann. Insbesondere bedeuten A¹ und A² Methyl COOA¹- ersetzt sein kann. Insbesondere bedeuten A¹ und A² Methyl oder Ethyl.

Hal bedeutet vorzugsweise F, Cl oder Br, aber auch I, insbesondere aber Cl.

Die Reste R¹ und R² können gleich oder verschieden sein und stehen vorzugsweise in der 3- und 4-Position des Phenylrings. Sie bedeuten beispielsweise jeweils unabhängig voneinander H, OH, OAlkyl, F, Cl, Br oder I oder zusammen Alkylen, wie z.B. Propylen, Butylen oder Pentylen, ferner Ethylenoxy, Methylendioxy oder Ethylendioxy. Bevorzugt stehen sie auch jeweils für Alkoxy, wie z.B. für Methoxy, Ethoxy oder Propoxy, ferner für Hydroxy.

Der Begriff "Heteroaromat" steht vorzugsweise für Pyridyl, Pyrazolyl, lmidazolyl, Oxazolyl, Isoxazolyl, Thiazolyl, Triazolyl, Chinolinyl, Isochinolinyl, Acridinyl, Pyridazyl, Pyrimidyl, Pyrazinyl, Phenazinyl, 9H-Purinyl, Pteridyl, insbesondere für 2,3 oder 4-Pyridyl, Pyridazyl, Pyrimidyl, Pyrazinyl, Pyrazolyl und Imidazolyl.

Für die gesamte Erfindung gilt, daß sämtliche Reste, die mehrfach auftreten, gleich oder verschieden sein können, d.h. unabhängig voneinander sind.
Dementsprechend ist Gegenstand der Erfindung insbesondere die Verwendung derjenigen Verbindungen der Formel I, in denen mindestens einer der genannten Reste eine der vorstehend angegebenen bevorzugten Bedeutungen hat.

Besonders bevorzugte Verbindungen der Formel I sind die der Verbindungen I1 bis 19: 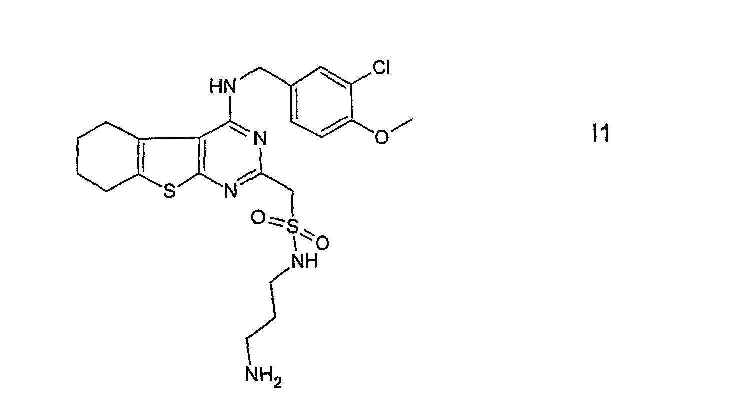 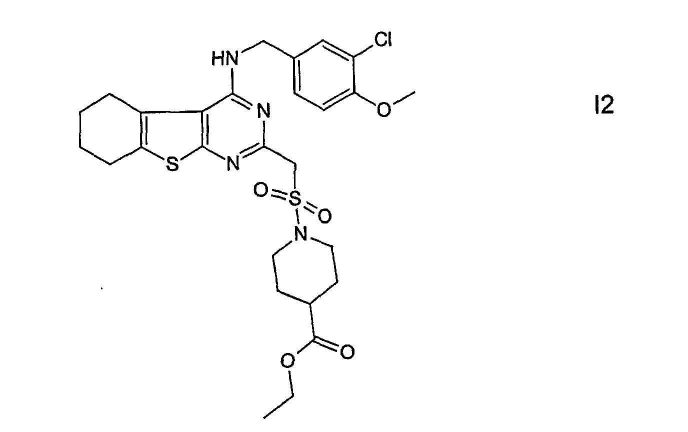 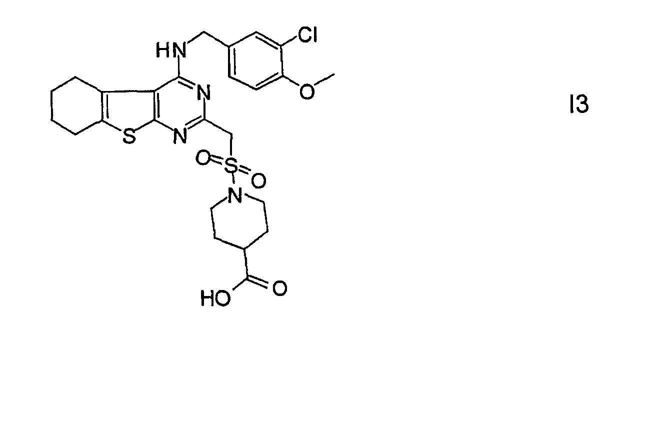 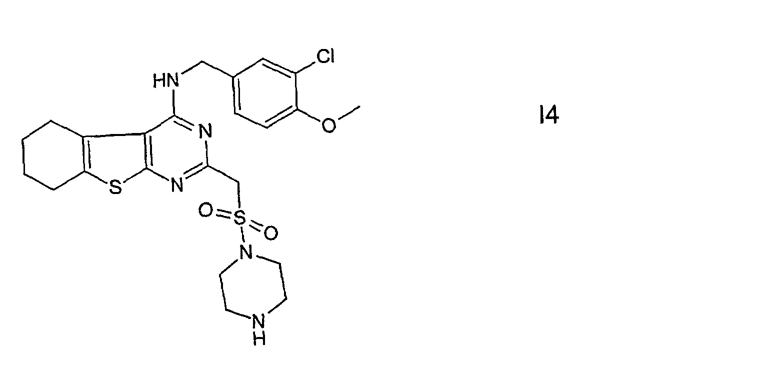 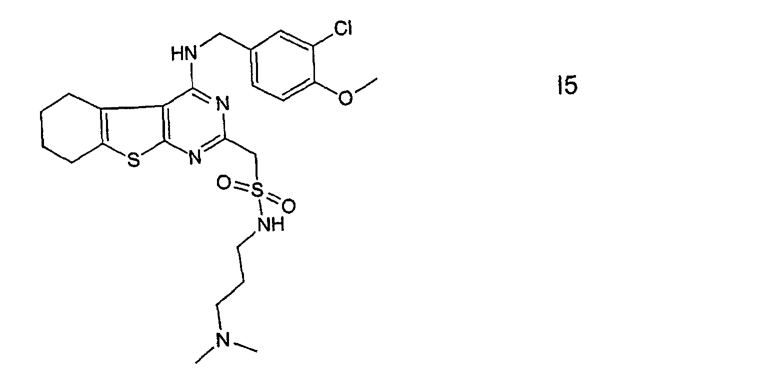 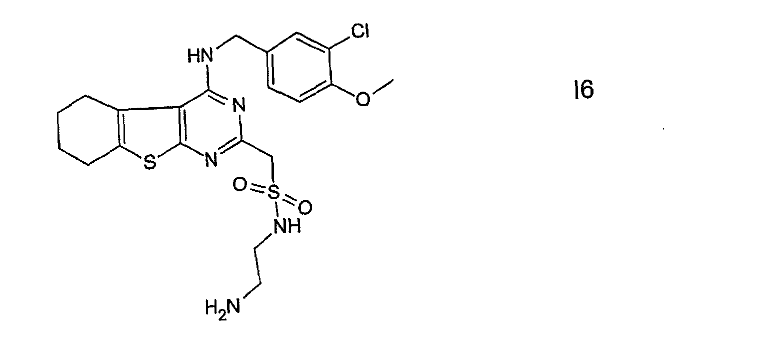 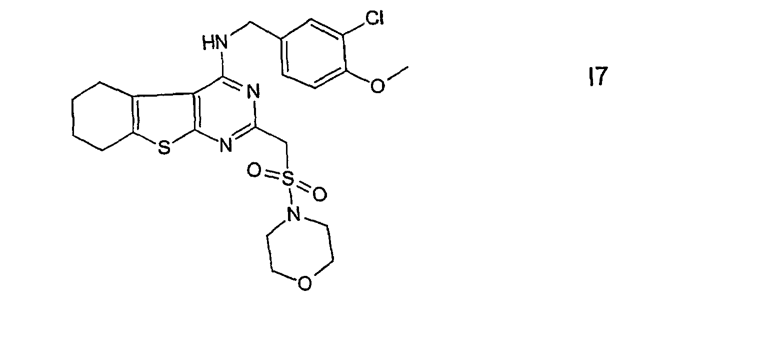 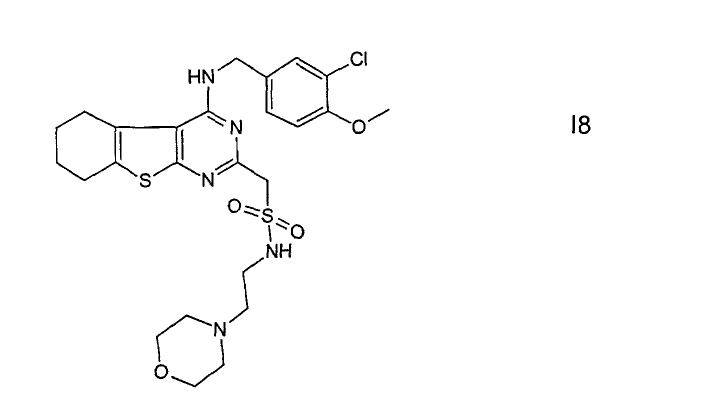 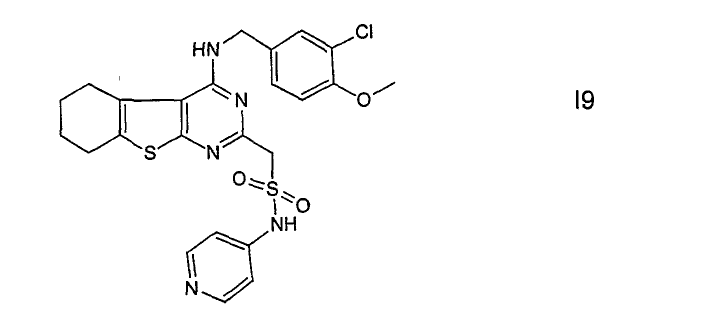 sowie deren Salze und Solvate. Von Verbindung 13 ist insbesondere das Ethanolammonium-Salz bevorzugt.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart), beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

In den Verbindungen der Formeln II und III haben R¹, R² und X die angegebenen Bedeutungen, insbesondere die angegebenen bevorzugten Bedeutungen.

Falls L eine reaktionsfähige veresterte OH-Gruppe bedeutet, so ist diese vorzugsweise Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy, ferner auch 2-Naphthalinsulfonyloxy).

Die Verbindungen der Formel I können vorzugsweise erhalten werden, indem man Verbindungen der Formel II 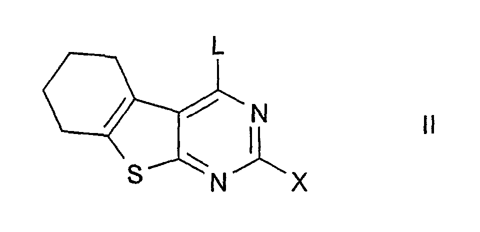 worin
X die angegebene Bedeutung hat,
und L Cl, Br, OH, SCH₃ oder eine reaktionsfähige veresterte OH-Gruppe bedeutet,
   mit Verbindungen der Formel III 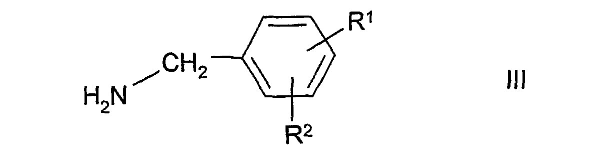 worin
R¹ und R² die angegebenen Bedeutungen haben,
   umsetzt
   oder
   eine Verbindung der Formel IV 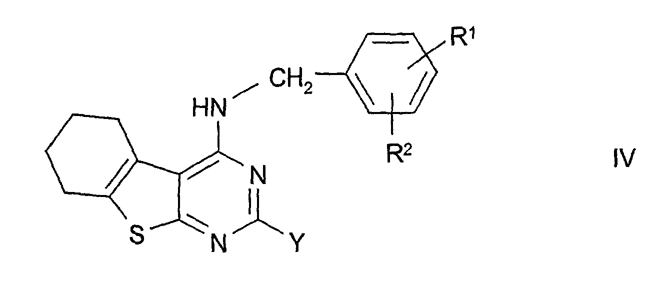 worin
   - Y: -CH₂SO₂Q,
   - Q: Cl, Br, OH, oder eine reaktionsfähige veresterte OH-Gruppe bedeutet
   und
R¹ und R² die in Anspruch 1 angegebene Bedeutung aufweisen, mit einer Verbindung der Formel V

   HNR³R⁴ V

   worin
R³ und R⁴ die in Anspruch 1 angegebenen Bedeutungen haben,
   umsetzt,
   oder
   und/oder daß man eine Verbindung der Formel I in eines ihrer Salze überführt.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt. Andererseits ist es möglich, die Reaktion stufenweise durchzuführen.

Die Ausgangsverbindungen der Formel II und III sind in der Regel bekannt. Sind sie nicht bekannt, so können sie nach an sich bekannten Methoden hergestellt werden.

Verbindungen der Formel II können z.B. durch Umsetzung mit POCl₃ aus den entsprechenden Hydroxypyrimidinen erhalten werden, die aus Thiophenderivaten und CN-substituierten Alkylencarbonsäureestern aufgebaut werden (Eur. J. Med. Chem. 23, 453 (1988)).
Die Darstellung der Hydroxypyrimidine erfolgt nach der für die Herstellung von Pyrimidinderivaten üblichen Cyclisierung von 2-Aminothiophen-3-carbonsäure-derivaten mit Aldehyden oder Nitrilen (z.B. Houben Weyl E9b/2).

Im einzelnen erfolgt die Umsetzung der Verbindungen der Formel II mit den Verbindungen der Formel III in Gegenwart oder Abwesenheit eines inerten Lösungsmittels bei Temperaturen zwischen etwa -20 und etwa 150°, vorzugsweise zwischen 20 und 100°.

Der Zusatz eines säurebindenden Mittels, beispielsweise eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylamin, Pyridin oder Chinolin oder eines Überschusses der Aminkomponente kann günstig sein.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, N-Methylpyrrolidon oder Dimethylformamid (DMF); Nitrile wie Acetonitril; Sulfoxide wie Dimethylsulfoxid (DMSO); Nitroverbindungen wie Nitromethan oder Nitrobenzol; Ester wie Ethylacetat oder Gemische der genannten Lösungsmittel.

Es ist ferner möglich, in einer Verbindung der Formel I einen Rest R¹ oder R² in einen anderen Rest R¹ oder R² umzuwandeln., z.B. indem man einen Ester zu einer OH-Gruppe hydrolysiert.
Estergruppen können z.B. mit NaOH oder KOH in Wasser, Wasser-THF oder Wasser-Dioxan bei Temperaturen zwischen 0 und 100° verseift werden.
Ether können druch Alkylierung der erhaltenen Hydroxygruppen unter Standardbedingungen erhalten werden.

Eine Säure der Formel I kann mit einer Base in das zugehörige Säureadditionssalz übergeführt werden, beispielsweise durch Umsetzung äquivalenter Mengen der Säure und der Base in einem inerten Lösungsmittel wie Ethanol und anschließendes Eindampfen. Für diese Umsetzung kommen insbesondere Basen in Frage, die physiologisch unbedenkliche Salze liefern.
So kann die Säure der Formel I mit einer Base (z.B. Natrium- oder Kaliumhydroxid oder -carbonat) in das entsprechende Metall-, insbesondere Alkalimetall- oder Erdalkalimetall-, oder in das entsprechende Ammoniumsalz umgewandelt werden.
Für diese Umsetzung kommen insbesondere auch organische Basen in Frage, die physiologisch unbedenkliche Salze liefern, wie z.B. Ethanolamin.

Andererseits kann eine Base der Formel I mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und -disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Gegenstand der Erfindung sind ferner pharmazeutische Zubereitungen, enthaltend mindestens eine Verbindung der Formel I und/oder eines ihrer physiologisch unbedenklichen Salze und/oder Solvate zur zur Behandlung von Angina, Bluthochdruck, pulmonalem Hochdruck, congestivem Herzversagen, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose und zur Behandlung weiblicher Impotenz.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den neuen Verbindungen nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und /oder mehrere weitere Wirkstoffe enthalten, z.B. ein oder mehrere Vitamine.

Die Verbindungen der Formel I und ihre physiologisch unbedenklichen Salze können bei der Behandlung und Prophylaxe von Krankheiten eingesetzt werden, die durch einen zu geringen cGMP(cyclo-Guanosin-monophosphat)-Spiegel hervorgerufen werden und/oder durch eine Erhöhung des cGMP-Spiegels beeinflußt werden können. Durch den erhöhten cGMP-Spiegel wird eine Entzündungshemmung oder -verhinderung und Muskelentspannung bewirkt. Insbesondere können die erfindungsgemäßen Verbindungen bei der Behandlung von Angina, Bluthochdruck, pulmonalem Hochdruck, congestivem Herzversagen, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose und zur Behandlung weiblicher Impotenz eingesetzt werden.

Gegenstand der Erfindung ist die Verwendung der Verbindungen der Formel I sowie deren physiologisch unbedenklichen Salze und/oder Solvate zur Herstellung eines Arzneimittels zur Behandlung und Prophylaxe von Potenzstörungen, wie z.B. erektiler Dysfunktion, zur Behandlung und Prophylaxe von Angina, Bluthochdruck, pulmonalem Hochdruck, congestivem Herzversagen, Herzinfarkt, chronischer obstruktiver pulmonaler Krankheit (COPD), Cor pulmonale, Rechtsherzinsuffizienz, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose, zur Behandlung weiblicher Sexualstörungen, Entzündungen, Osteoporose, zur Behandlung von maligner Hypertonie, Phäochromazytom, peripherer Gefäß(-verschluß)-erkrankungen, Gefäßerkrankungen, Thrombozytopenie, Ulcus pepticum, Darmbewegungsstörungen, perkutaner transluminaler Koronarangioplastie, Karotis Angioplastie, postoperativer Stenose des Koronarbypasses, Vorwehen und benigner Prostata-Hyperplasie.

Dabei werden die Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Gegenstand der Erfindung sind ferner Arzneimittel enthaltend mindestens eine Verbindung der Formel I und/oder ihre physiologisch verträglichen Salze und Solvate, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer physiologisch verträglichen Salze und Solvate, und
(b) einer wirksamen Menge eines weiteren Arzneimittetwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer physiologisch verträglichen Salze und Solvate
und einer wirksamen Menge eines weiteren Arzneimittelwirkstoffs gelöst oder in lyophylisierter Form vorliegt.

Gegenstand der Erfindung ist ferner die Verwendung von Verbindungen der Formel I und/oder ihrer physiologisch verträglichen Salze und Solvate, zur Herstellung eines Arzneimittels zur Behandlung von Krankheiten des Herz-Kreislaufsystems, zur Behandlung und/oder Prophylaxe von Potenzstörungen wie z.B. erektiler Dysfunktion, zur Behandlung und Prophylaxe von Angina, Bluthochdruck, pulmonalem Hochdruck, congestivem Herzversagen, Herzinfarkt, chronischer obstruktiver pulmonaler Krankheit (COPD), Cor pulmonale, Rechtsherzinsuffizienz, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose, zur Behandlung und Prophylaxe weiblicher Sexualstörungen, Entzündungen, Osteoporose, weiterhin zur Behandlung von maligner Hypertonie, Phäochromazytom (katecholaminproduzierender Tumor der Nebennierenrinde), bei peripheren Gefäß(-verschluß)-erkrankungen, Gefäßerkrankungen, Thrombozytopenie, Ulcus pepticum (gutartiges Darmgeschwür), Darmbewegungsstörungen, perkutaner transluminaler Koronarangioplastie, Karotis Angioplastie, postoperativer Stenose des Koronarbypasses, Vorwehen sowie benigner Prostata-Hyperplasie, in Kombination mit mindestens einem weiteren Arzneimittelwirkstoff.

Die erfindungsgemäßen Verbindungen der Formel I können zusammen mit anderen Wirkstoffen verwendet werden, wie z.B. mit Vasodilatoren, α-adrenergen Inhibitoren, wie z.B. Phentolamin, Prazocin oder Yohimbin, gemischten α,β-Inhibitoren, wie z.B. Carvedilol, Prostaglandin El und Prostacyclin, ACE (Angiotensin Converting Enzyme) - Hemmern, NEP (Neutrale Endopeptidase) - Hemmern, zentral wirksamen dopaminergen Wirkstoffen, wie z.B. Apomorphin, vasoaktiven Intestinalpeptiden, Calciumkanalblockern und Verbindungen wie Thiaziden.

Gegenstand der Erfindung sind daher pharmazeutische Formulierungen enthaltend ein Prostaglandin oder Prostaglandinderivat und mindestens eine Verbindung der Formel I.
Bevorzugt sind Prostaglandine oder Prostaglandinderivate ausgewählt aus der Gruppe PGE₀, PGA₁, PGB₁, PGF_{1α}, PGA₂, PGB₂, 19-Hydroxy-PGA₁, 19-Hydroxy-PGB₁, 19-Hydroxy-PGA₂, 19-Hydroxy-PGB₂, PGE₃, PGF_{3α}, Alprostadil (PGE₁), Dinoprost (PGF₂), Dinoprostone (PGE₂), Epoprostenol Natrium (PGI₂; Prostacyclin Natrium), Gemeprost, Iloprost, Latanoprost, Misoprostol, Sulprostone, Carboprost Thromethamin, Dinoprost Thromethamin, Lipoprost, Metenoprost, Tiaprost.

Bevorzugt sind besonders Prostaglandine oder Prostaglandinderivate ausgewählt aus der Gruppe Alprostadil (PGE₁), Dinoprost (PGF₂), Dinoprostone (PGE₂), Epoprostenol Natrium (PGI₂; Prostacyclin Natrium), Gemeprost, Iloprost, Latanoprost, Misoprostol, Sulprostone, Carboprost Thromethamin, Dinoprost Thromethamin, Lipoprost, Metenoprost, Tiaprost.

Besonders bevorzugt ist PGE₁ oder Prostacyclin, insbesondere bevorzugt ist Prostacyclin.

Gegenstand der Erfindung sind ferner pharmazeutische Formulierungen enthaltend einen Calcium-Antagonisten und mindestens eine Verbindung der Formel I.
Bevorzugt sind Calcium-Antagonisten ausgewählt aus der Gruppe der selektiven und nicht-selektiven Calcium-Antagonisten.

Bevorzugt sind selektive Calcium-Antagonisten ausgewählt aus der Gruppe der Dihydropyridinderivate, Phenylalkylaminderivate, Benzothiazepinderivate und anderen selektiven Calcium-Antagonisten.

Dihydropyridinderivate sind vorzugsweise ausgewählt aus der Gruppe Amlodipine, Felodipine, Isradipine, Nicardipine, Nifedipine, Nimodipine, Nisoldipine, Nitrendipine, Lacidipine, Nilvadipine, Manidipine, Barnidipine, Lercanidipine.

Die Phenylalkylaminderivate sind vorzugsweise ausgewählt aus der Gruppe Verapamil, Gallopamil.

Die Benzothiazepinderivate bedeuten vorzugsweise Diltiazem.

Die anderen selektiven Calcium-Antagonisten bedeuten vorzugsweise Mibefradil.

Die nicht-selektiven Calcium-Antagonisten sind vorzugsweise ausgewählt aus der Gruppe Fendiline, Bepridil, Lidoflazine, Perhexiline.

Gegenstand der Erfindung sind ferner pharmazeutische Formulierungen enthaltend ein Antithromboticum und mindestens eine Verbindung der Formel I.
Unter den Begriff Antithrombotica fallen auch sogenannte Antikoagulantien und Blutplättchenaggregationshemmer (Thrombozytenaggregationshemmer).
Bevorzugte Antithrombotica sind Vitamin K Antagonisten, Heparinverbindungen, Thrombozytenaggregationshemmer, Enzyme, Faktor Xa Inhibitoren, Faktor VIIa Inhibitoren und andere antithrombotische Agenzien.

Bevorzugte Vitamin K Antagonisten sind ausgewählt aus der Gruppe Dicoumarol, Phenindione, Warfarin, Phenprocoumon, Acenocoumarol, Ethyl-biscoumacetat, Clorindione, Diphenadione, Tioclomarol.

Bevorzugte Heparinverbindungen sind ausgewählt aus der Gruppe Heparin, Antithrombin III, Dalteparin, Enoxaparin, Nadroparin, Parnaparin, Reviparin, Danaparoid, Tinzaparin, Sulodexide.

Bevorzugte Thrombozytenaggregationshemmer sind ausgewählt aus der Gruppe Ditazole, Cloricromen, Picotamide, Clopidogrel, Ticlopidine, Acetylsalicylsäure, Dipyridamole, Calcium carbassalat, Epoprostenol, Indobufen, Iloprost, Abciximab, Tirofiban, Aloxiprin, Intrifiban.

Bevorzugte Enzyme sind ausgewählt aus der Gruppe Streptokinase, Alteplase, Anistreplase, Urokinase, Fibrinolysin, Brinase, Reteplase, Saruplase.

Bevorzugte Antithrombotica sind weiterhin die Blutplättchen-Glycoprotein-Rezeptor (IIb/IIIa)-Antagonisten, die die Blutplättchenaggregation inhibieren.
Bevorzugte Verbindungen sind z.B. beschrieben in EP 0 623 615 B1 auf Seite 2 oder in der EP 0 741 133 A2 Seite 2, Zeile 2 bis Seite 4 Zeile 56.

Bevorzugte Faktor Xa- und Vlia-Inhibitoren sind z.B. die in WO 9916751, WO 9931092, WO 9957096, WO 0012479, WO 0020416, WO 0040583, WO 0051989 beschriebenen Verbindungen der Formel I.
Andere bevorzugte Faktor Xa Inhibitoren sind z.B. die in den nachstehenden Dokumenten beschriebenen Verbindungen:
a) in WO 97/30971, Seite 4, Zeile 5 bis Seite 13, Zeile 19;
b) in EP 0 921 116 A1, Seite 2, Zeile 1 bis Zeile 51;
c) in EP 0 540 051 B1, Seite 2, Zeile 41 bis Seite 3, Zeile 14;
d) in EP 0 798 295 A1, Seite 69, Zeile 10 bis Seite 71, Seite 53;

Bevorzugte andere Verbindungen sind ausgewählt aus der Gruppe Defibrotide, Desirudin oder Lepirudin.

Gegenstand der Erfindung sind auch solche pharmazeutischen Formulierungen enthaltend einen Endothelin-Rezeptor-Antagonisten und mindestens eine Verbindung der Formel I.

Bevorzugte Endothelin-Rezeptor-Antagonisten sind Bosentan, Tezosentan und Sitaxentan (TBC-11251; J.Med.Chem., 40, No.11, 1690-97, 1997). Bevorzugte Endothelin-Rezeptor-Antagonisten sind somit weiterhin
a) BMS-193884 (EP 558258),
b) BMS-207940 (Pharmaprojects (13.06.97)),
c) BQ-123 (Exp.Opin.Invest.Drugs, 1997, 6, No.5,475-487),
d) SB-209670 (Exp.Opin.Invest.Drugs, 1997, 6, No.5, 475-487),
e) SB-217242 (Exp.Opin.Invest.Drugs, 1997, 6, No.5, 475-487),
f) SB-209598 (Trends in Pharmacol. Sci., 17, 177-81,1996),
g) TAK-044 (Exp.Opin.Invest.Drugs, 1997,6, No.5, 475-487),
h) Bosentan (Trends in Pharmacol. Sci., 18, 408-12, 1997),
i) PD-156707 (J.Med.Chem., 40, No.7, 1063-74,1997),
j) L-749329 (Bioorg.Med.Chem.Lett., 7, No.3, 275-280, 1997),
k) L-754142 (Exp.Opin.lnvest.Drugs, 1997, 6, No.5, 475-487),
I) ABT-627 (J.Med.Chem., 40, No.20, 3217-27,1997),
m) A-127772 (J.Med.Chem., 39, No.5, 1039-1048, 1996),
n) A-206377 (213^{th} American Chemical Society National Meeting, San Francisco, California, USA, 13 - 17 April 1997, Poster, MEDI 193),
o) A-182086 (J.Med.Chem., 40, No.20, 3217-27, 1997),
p) EMD-93246 (211^{th} American Chemical Society National Meeting, New Orleans, USA, 1996, Poster, MEDI 143),
q) EMD-122801 (Bioorg.Med.Chem.Lett., 8, No.1, 17-22, 1998),
r) ZD-1611 (Trends in Pharmacol. Sci., 18, 408-12, 1997),
s) AC-610612 (R&D Focus Drug News (18.05.98)),
t) T-0201 (70^{th} Annual Meeting of the Japanese Pharmacological Society, Chiba, Japan, 22-15 March 1997, Lecture, O-133),
u) J-104132 (R&D Focus Drug News (15.12.97)),

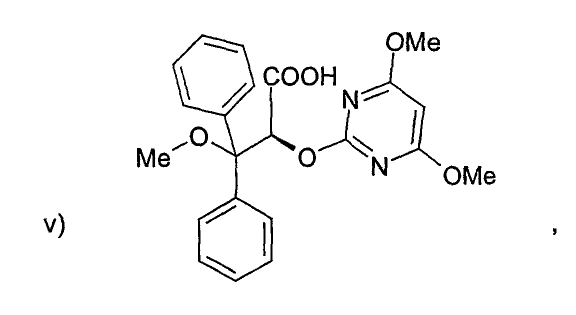

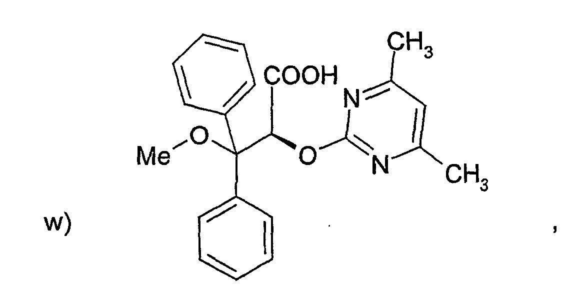

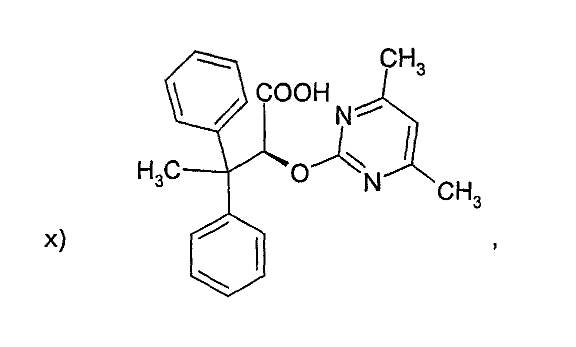

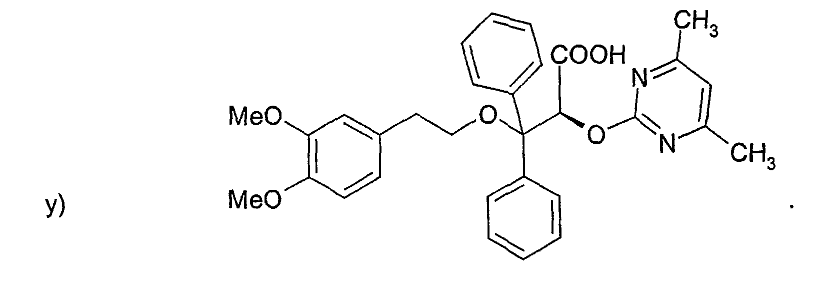

Besonders bevorzugte Endothelin-Rezeptor-Antagonisten sind z.B. die in EP 0733626, EP 0733626, EP 0755934, EP 0757039, EP 0796250, WO 9719077, WO 9730982, WO 9730996, DE 19609597, DE 19612101, WO 9827091, WO 9827077, WO 9841515, WO 9841521, WO 9842702, WO 9842709 oder WO 9905132 beschriebenen Verbindungen der Formel I.

Gegenstand der Erfindung sind weiterhin pharmazeutischen Formulierungen enthaltend einen Vasodilator, wie z.B. ein Nitrat, und mindestens eine Verbindung der Formel I.

Gegenstand der Erfindung sind vorzugsweise pharmazeutische Formulierungen enthaltend mindestens eine Verbindung der Formel I und einen Vasodilator, wie z.B. (a) ein organisches Nitrat, z.B. Nitroglycerin, Isosorbidmononitrat, Isosorbiddinitrat, Pentaerythrityltetra-, Pentaerythrityltri-, Pentaerythrityldi-, Pentaerythritylmononitrat, Propatylnitrat, Trolnitrat, Nicroandil, Mannitolhexanitrat, Inositolhexanitrat, N-[3-Nitratopivaloyl]-L-cycsteinethylester, (b) ein organisches Nitrit, z.B. Isoamylnitrit, (c) ein Thionitrit, (d) ein Thionitrat, (e) ein S-Nitrosothiol, wie z.B. S-Nitroso-N-acetyl-D,L-penicillamin, (f) Nitrosoproteine, (g) substituierte Furoxane, wie z.B. 1,2,5-Oxadiazol-2-oxide oder Furazan-N-oxide, (h) substituierte Sydnonimine, wie z.B. Moisidomine oder Mesocarb, (i) komplexe Nitrosylverbindungen, wie z.B. Eisennitrosylverbindungen, vorzugsweise Natriumnitroprussid oder (j) Stickoxid NO, das inhaliert wird.

Bevorzugte Vasodilatoren sind Nitrate ausgewählt aus der Gruppe Pentaerythrityltetra-, Pentaerythrityltri-, Pentaerythrityldi-, Pentaerythritylmononitrat, Isosorbidmononitrat, Isosorbiddinitrat, Glyceroltrinitrat.

Bevorzugt sind besonders Nitrate ausgewählt aus der Gruppe Pentaerythrityltetranitrat, Isosorbidmononitrat, Isosorbiddinitrat, Glyceroltrinitrat, ganz besonders bevorzugt ist Pentaerythrityltetranitrat.

Gegenstand der Erfindung ist weiterhin die Verwendung einer pharmazeutischen Formulierung enthaltend mindestens eine Verbindung der Formel I und mindestens ein Antithromboticum zur Herstellung eines Arzneimittels zur Behandlung von pulmonalem Hochdruck, congestivem Herzversagen (CHF), chronischer obstruktiver pulmonaler Krankheit (COPD), Cor pulmonale und/oder Rechtsherzinsuffizienz.

α-Adrenerge Inhibitoren hemmen die Vasokonstriktion im Corpus Cavernosum. Da PDE V - Hemmer die Vasodilatation des gleichen Gewebes der glatten Muskulatur erhöht, können zur Behandlung von Potenzstörungen (erektiler Dysfunktion) vorzugsweise auch pharmazeutische Formulierungen verwendet werden, enthaltend mindestens eine Verbindung der Formel I und mindestens einen α-adrenergen Inhibitor, wie z.B. Phentolamin oder Prazocin oder mindestens einen zentral wirksamen dopaminergen Wirkstoff, wie z.B. Apomorphin.

Gegenstand der Erfindung ist daher weiterhin die Verwendung einer pharmazeutischen Formulierung enthaltend mindestens eine Verbindung der Formel I und mindestens einen α-adrenergen Inhibitor, wie z.B. Phentolamin oder Prazocin oder mindestens einen zentral wirksamen dopaminergen Wirkstoff, wie z.B. Apomorphin zur Herstellung eines Arzneimittels zur Behandlung von Potenzstörungen.

Gegenstand der Erfindung ist weiterhin die Verwendung einer pharmazeutischen Formulierung enthaltend mindestens eine Verbindung der Formel I und mindestens einen Calcium-Antagonisten zur Herstellung eines Arzneimittels zur Behandlung von pulmonalem Hochdruck, congestivem Herzversagen (CHF), chronischer obstruktiver pulmonaler Krankheit (COPD), Cor pulmonale und/oder Rechtsherzinsuffizienz.

Gegenstand der Erfindung ist weiterhin die Verwendung einer pharmazeutischen Formulierung enthaltend mindestens eine Verbindung der Formel I und mindestens ein Nitrat zur Herstellung eines Arzneimittels zur Behandlung von pulmonalem Hochdruck, congestivem Herzversagen (CHF), chronischer obstruktiver pulmonaler Krankheit (COPD), Cor pulmonale und/oder Rechtsherzinsuffizienz.

Gegenstand der Erfindung ist weiterhin die Verwendung einer pharmazeutischen Formulierung enthaltend mindestens eine Verbindung der Formel I und mindestens einen Endothelin-Rezeptor-Antagonisten zur Herstellung eines Arzneimittels zur Behandlung von pulmonalem Hochdruck, congestivem Herzversagen (CHF), chronischer obstruktiver pulmonaler Krankheit (COPD), Cor pulmonale und/oder Rechtsherzinsuffizienz.

Gegenstand der Erfindung ist weiterhin die Verwendung einer pharmazeutischen Formulierung enthaltend mindestens eine Verbindung der Formel I und mindestens ein Prostaglandin oder ein Prostaglandinderivat zur Herstellung eines Arzneimittels zur Behandlung von pulmonalem Hochdruck, congestivem Herzversagen (CHF), chronischer obstruktiver pulmonaler Krankheit (COPD), Cor pulmonale und/oder Rechtsherzinsuffizienz.

Besonders bevorzugter Gegenstand der vorliegenden Anmeldung sind demnach Arzneimittel enthaltend mindestens eine Verbindung der Formel I und/oder ihre physiologisch unbedenklichen Salze und Solvate und mindestens einen weiteren Arzneimitteiwirkstoff ausgewählt aus der folgenden Gruppe a) bis k):
a) Prostaglandin oder Prostaglandinderivat,
b) Calcium-Antagonist,
c) Antithromboticum,
d) Endothelin-Rezeptor-Antagonist,
e) Nitrat,
f) α-adrenerger Inhibitor,
g) zentral wirksamer dopaminerger Wirkstoff,
h) ACE-Hemmer,
i) NEP-Hemmer,
j) gemischter α,β-Inhibitor,
k) vasoaktives Intestinalpeptid.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation.

Massenspektrometrie (MS):
El (Elektronenstoß-lonisation) M⁺
FAB (Fast Atom Bombardment) (M+H)⁺

Gegenstand der Erfindung sind insbesondere die in den nachstehenden Beispielen aufgeführten Verbindungen der Formel I sowie deren physiologisch unbedenklichen Salze und/oder Solvate und deren Verwendung zur Herstellung eines Arzneimittels zur Behandlung von Angina, Bluthochdruck, pulmonalem Hochdruck, congestivem Herzversagen, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose und zur Behandlung weiblicher Impotenz.

### Beispiel 1

### Schritt A:

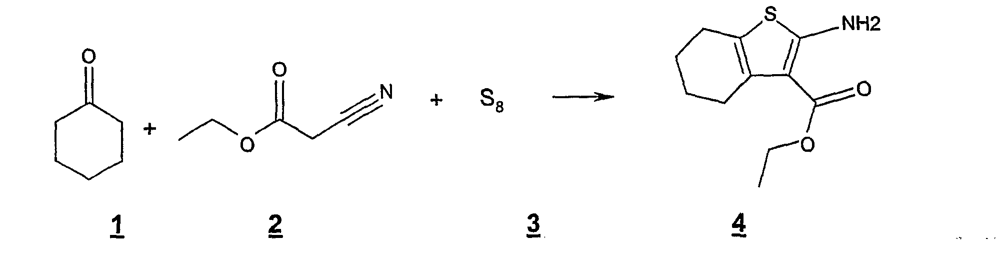

In eine Mischung von 300 ml **2**, 290 ml Cyclohexanon und 670 ml Ethanol wurden unter Rühren 99,0 g Schwefel eingetragen. Anschließend tropfte man bei etwa 50°C bis 65 °C 275 ml Diethylamin hinzu und kristallisierte den entstandenen Niederschlag nach Filtration aus Ethanol um, wodurch **4** erhalten wurde.

### Schritt B:

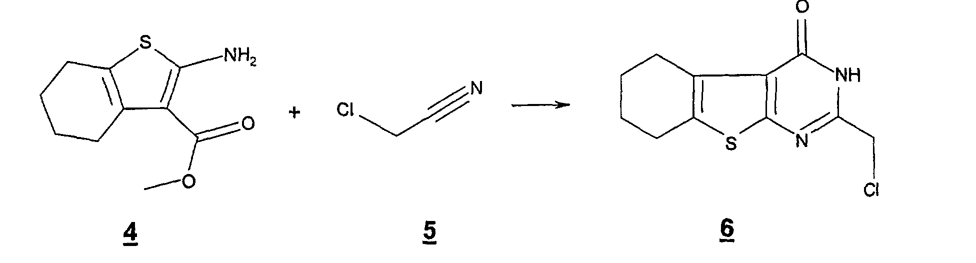

In eine Lösung von 350,0 g **4** und 132 ml Chloracetonitril in 2 I Dioxan wurden innerhalb von 3 h unter Rühren ca. 500 g HCl eingeleitet, wobei die Temperatur bei 45 °C gehalten wird. Nach Entfernung des Lösungsmittels, Alkalisierung mit Bicarbonat-Lösung und Filtration wurde **6** in kristalliner Form erhalten.

### Schritt C:

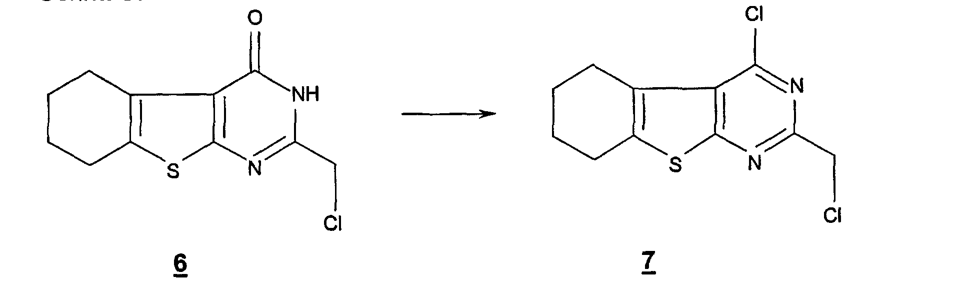

In eine Mischung von 1,00 I Thionylchlorid mit 123,116 ml N, N-Dimethylformamid wurden 400,0 g **6** bei 25-30 °C eingetragen. Anschließend wurde über Nacht bei RT gerührt und wie üblich auf-gearbeitet, wodurch **7** in kristalliner Form erhalten wurde.

### Schritt D:

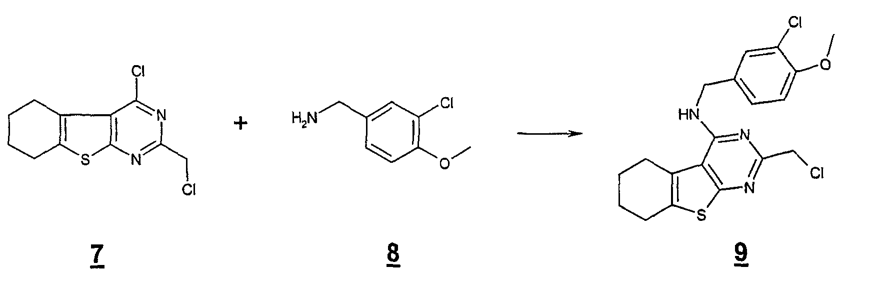

384,0 g **7** wurden in 4 I THF vorgelegt und 514,88 g **8** gelöst in 1 I THF unter leichter Eiswasser-kühlung bei 20°C zügig zugetropft. Nach Rühren über Nacht arbeitete man wie üblich auf und erhielt hierdurch **9** in Form von Kristallen (Fp. 120 - 121°C)

### Schritt E:

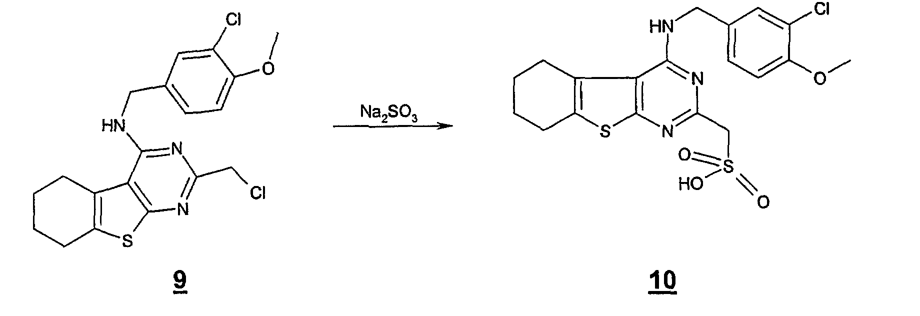

Zu einer Lösung von 15,00 g **9** in 60 ml Dioxan wurden 100 ml einer gesättigten Lösung von Natriumsulfit in Wasser gegeben. Durch Rühren über Nacht und üblicher Aufarbeitung wurde **10** erhalten.

### Schritt F:

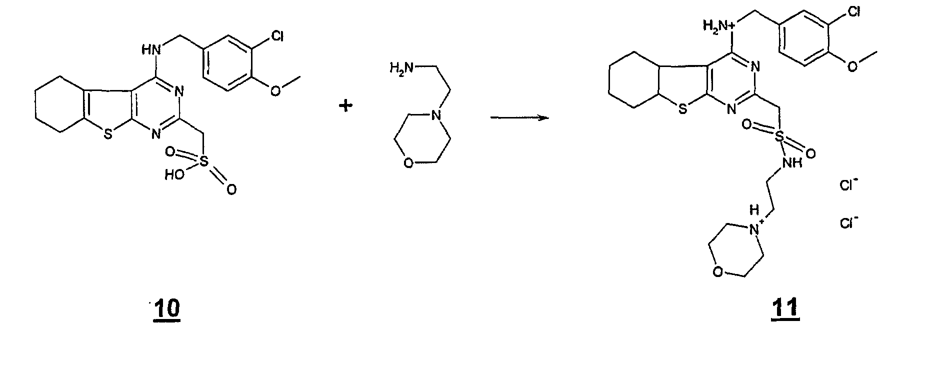

Eine Mischung von 1,140 g **10** in 7,50 ml Phosphorylchlorid wurde unter Rühren mit 0,4 ml Wasser versetzt und für 15 Min. bei 60°C gerührt. Nach Zugabe von 6,80 g Phosphorpentachlorid und 30-minütigem Rühren wurde alles Flüchtige entfernt und der Rückstand in 30 ml Dichlormethan bei max. 10°C zu 4,00 g N-(2-Aminoethyl)-morpholin in 30 ml Dichlormethan getropft. Nach 3 h schüttelte man die Mischung mit 1 N HCl aus und filtrierte die ausgefallenen Kristalle der Verbindung **11** ab.

Analog werden unter Verwendung der entsprechenden Vorstufen die folgenden Verbindungen erhalten:

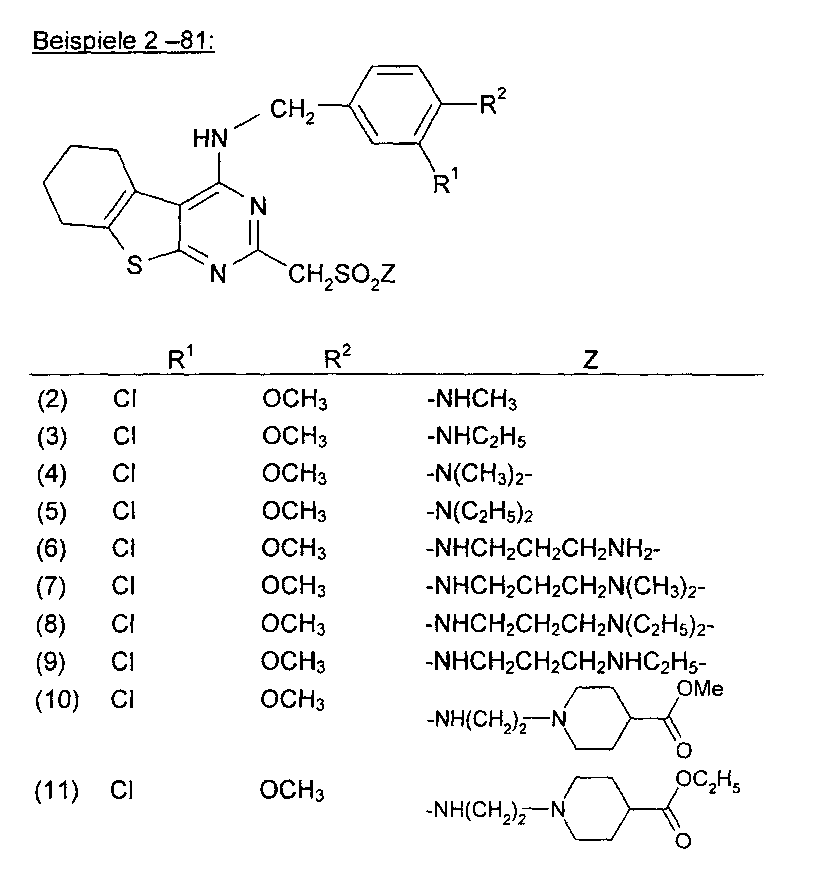

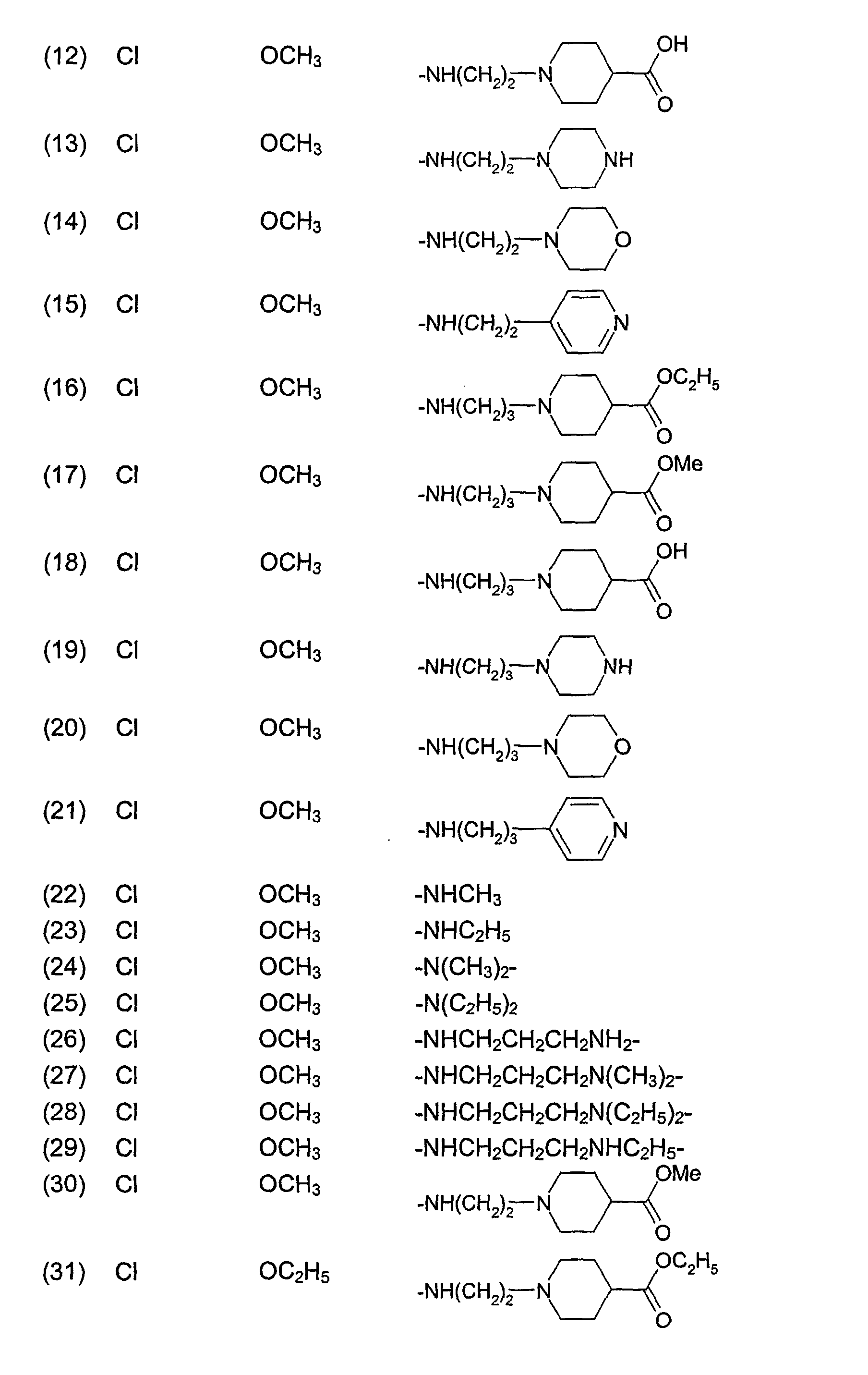

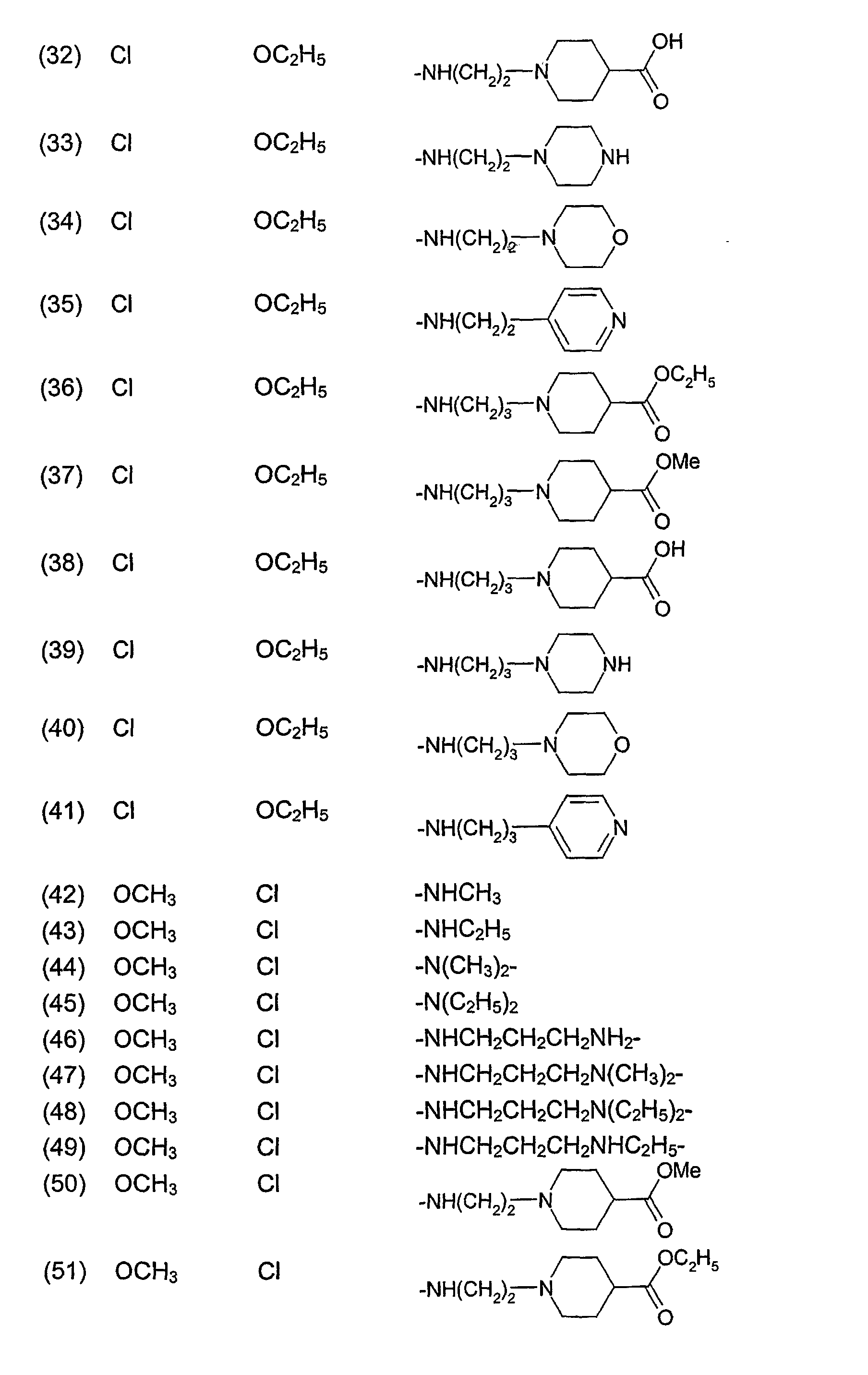

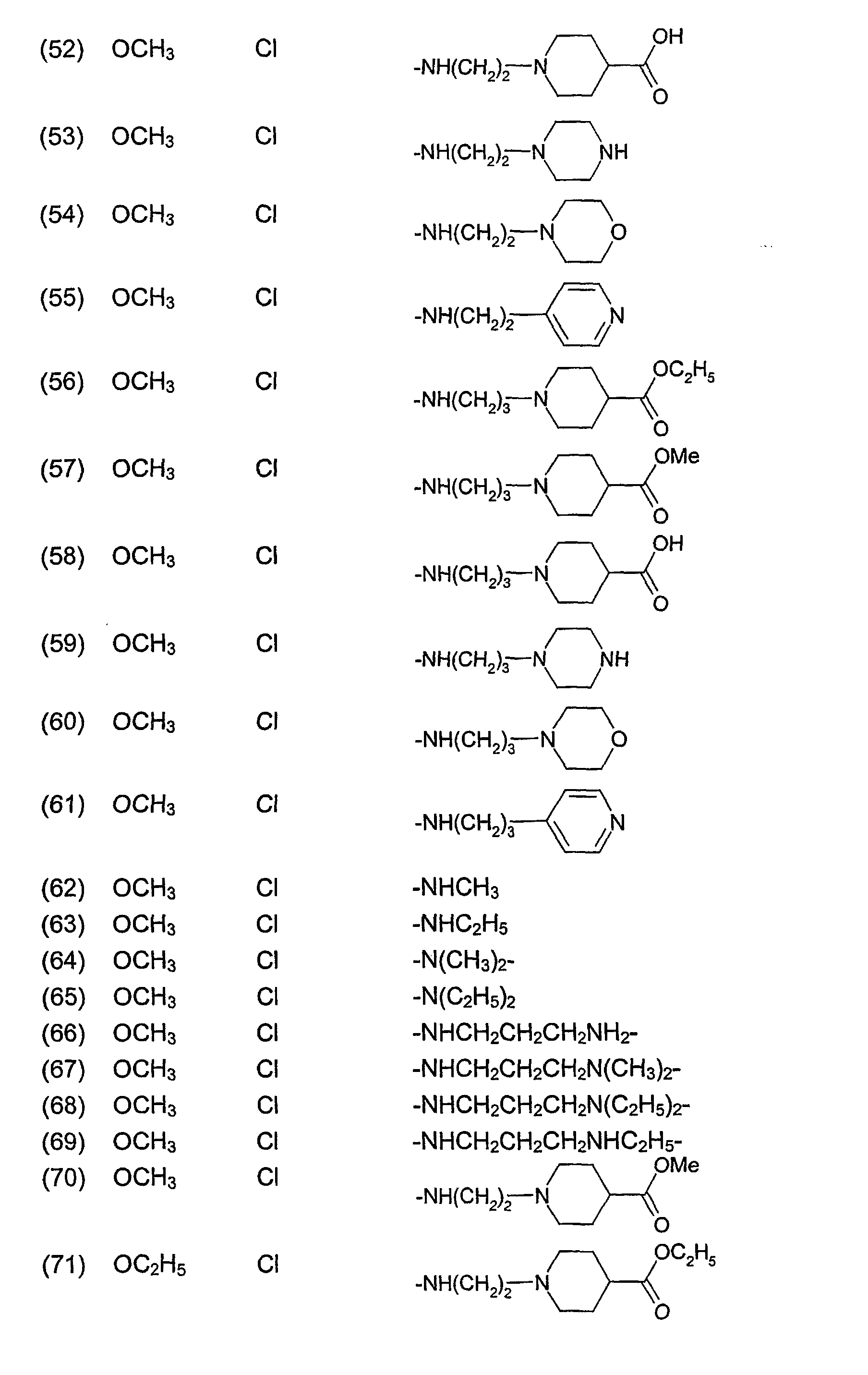

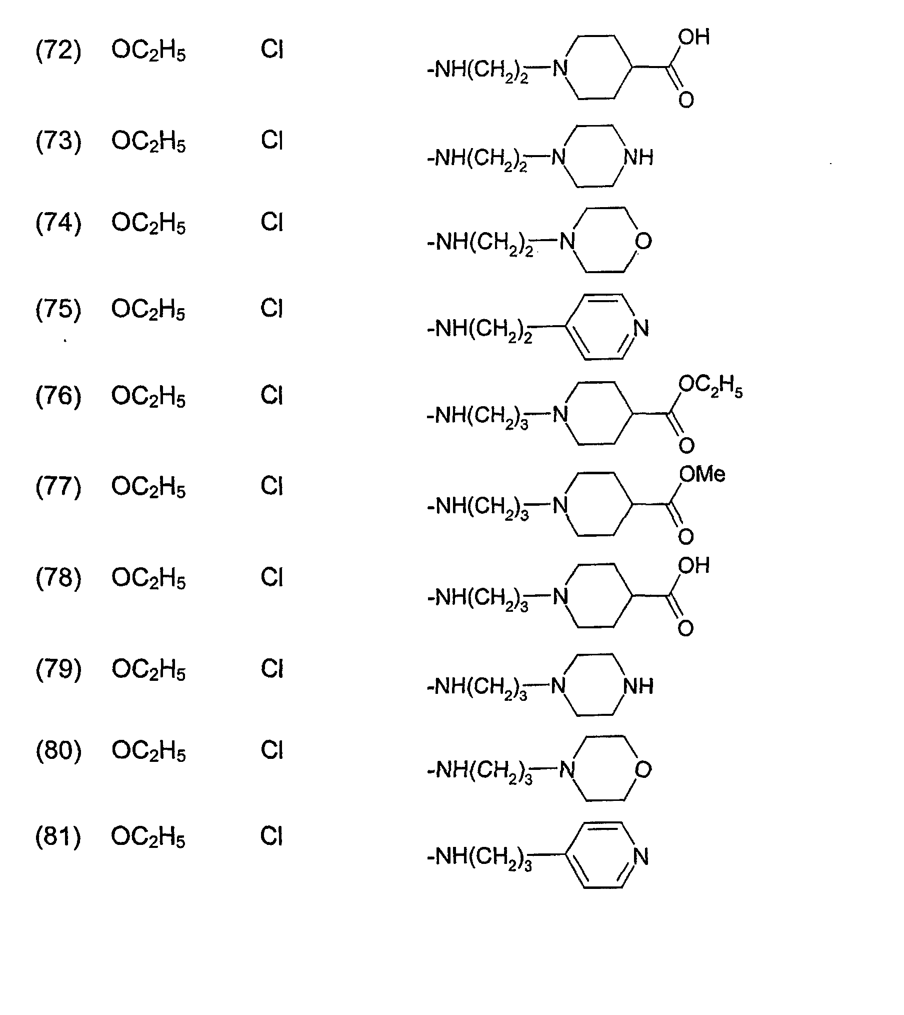

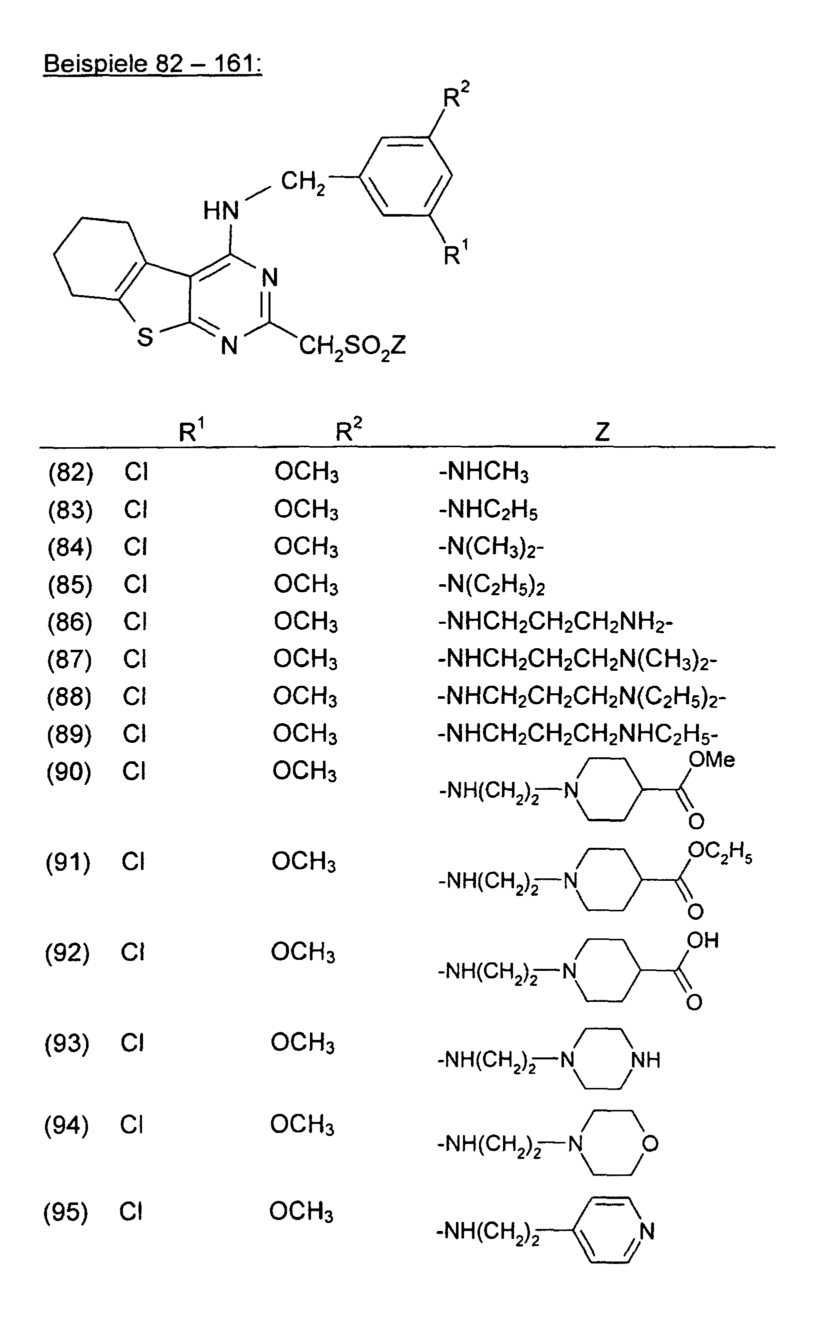

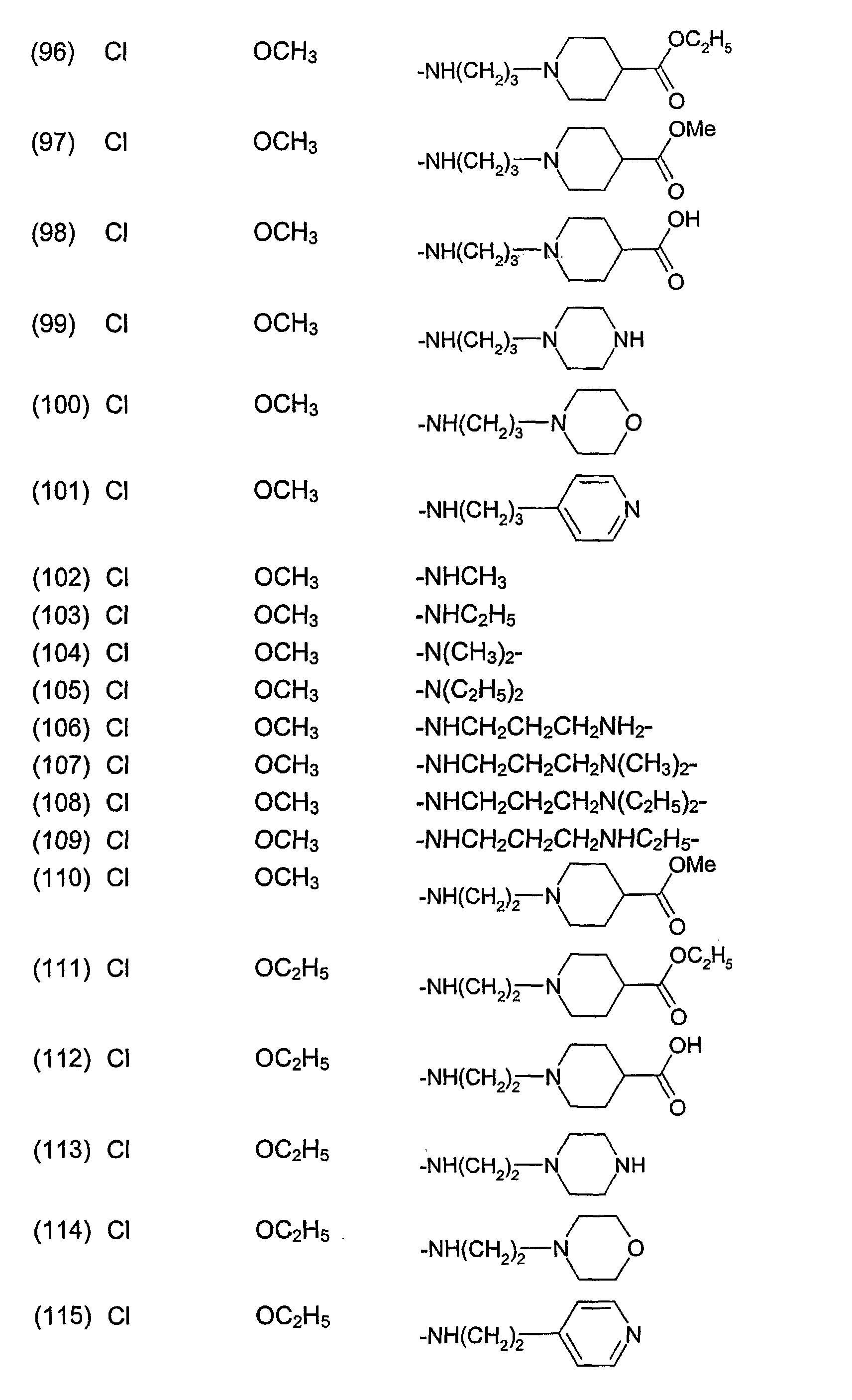

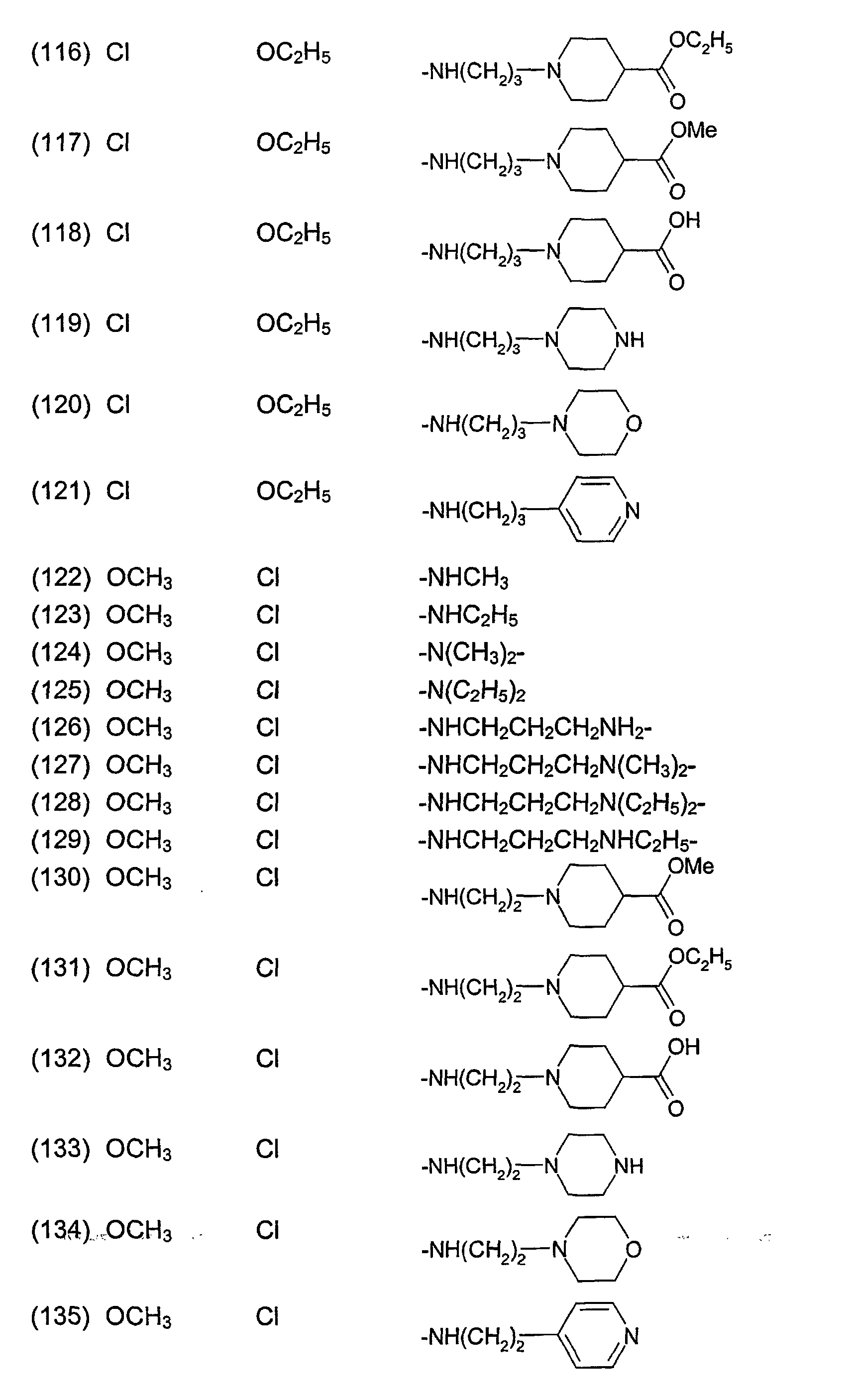

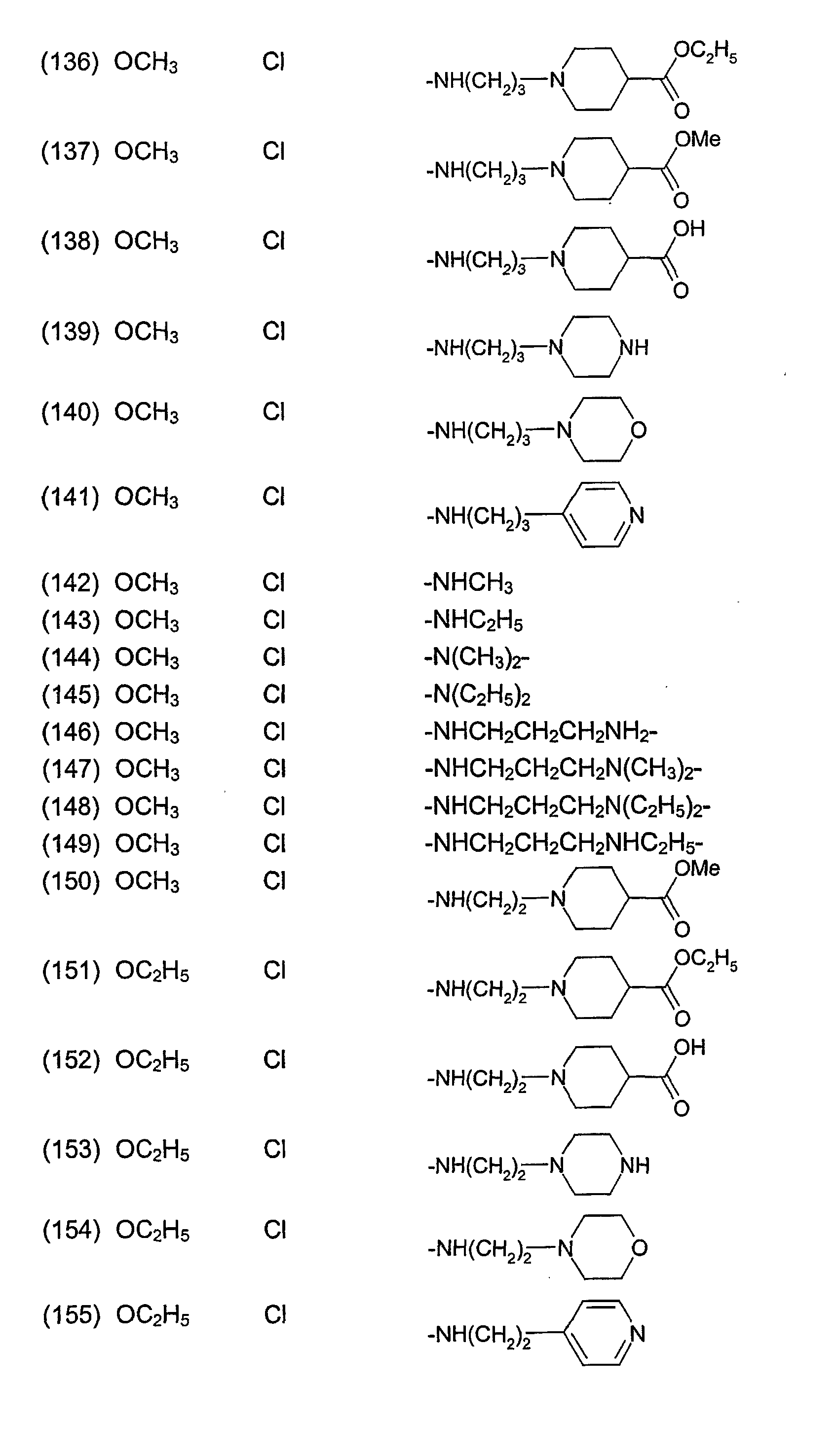

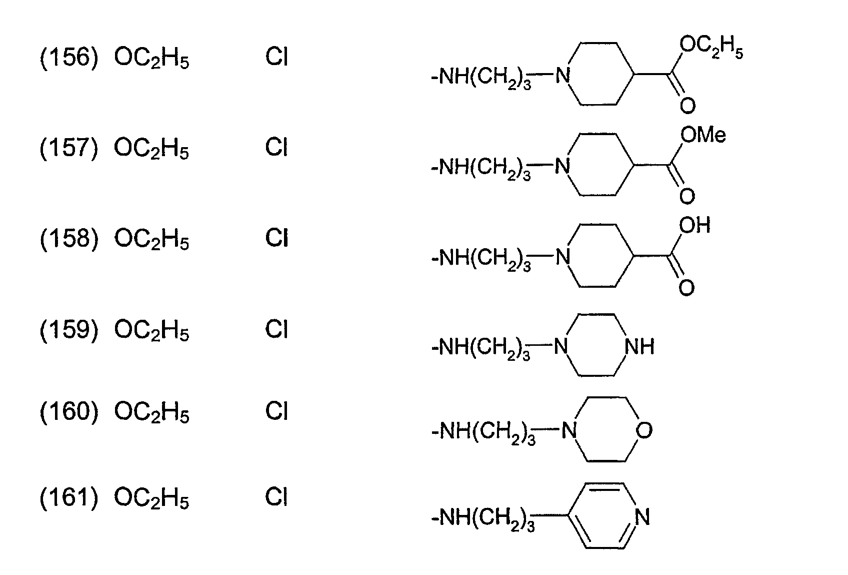

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

### Beispiel I: Inhalations-Spray

Man löst 14 g Wirkstoff der Formel I in 10 I isotonischer NaCl-Lösung und füllt die Lösung in handelsübliche Sprühgefäße mit Pump-Mechanismus. Die Lösung kann in Mund oder Nase gesprüht werden. Ein Sprühstoß (etwa 0,1 ml) entspricht einer Dosis von etwa 0,14 mg.

## Patentansprüche

1. Verbindungen der Formel I 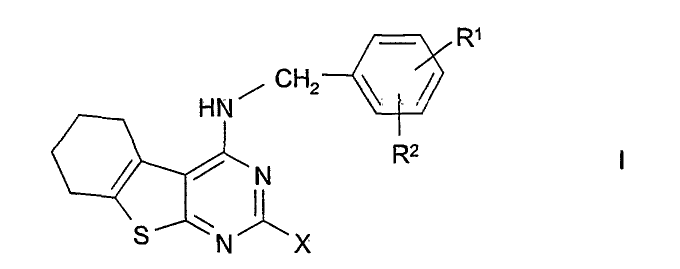 worin
R¹, R² jeweils unabhängig voneinander H, OA¹, OCOA¹, OH oder Hal,
wobei R¹ und R²zusammen auch Alkylen mit 3-5 C-Atomen, -O-CH₂-CH₂-, -CH₂-O-CH₂-, -O-CH₂-O- oder -O-CH₂-CH₂-O-,
X -CH₂SO₂NR³R⁴,
R³, R⁴ unabhängig voneinander H, ein Heteroaromat, unsubstituiertes oder terminal durch -NH₂, NHA¹ oder -NA¹A² substituiertes Alkyl oder Alkenyl mit 1-10 C-Atomen, worin eine oder zwei CH₂-Gruppen durch -CH=CH-Gruppen, -O-, -NH- oder -NA¹- ersetzt sein können, wobei R³ und R⁴ zusammen auch unsubstituiertes oder ein- oder mehrfach durch -Hal, -NH₂, -NHA, -NA¹A², -NHCOA¹, NHCOOA¹, -COOH, -COOA¹, -CONH₂, -CONHA¹, oder -CONA¹A² substituiertes Cycloalkyl oder Cycloalkylen mit 3-7 C-Atomen, worin eine oder zwei -CH₂-Gruppen durch -O-, -NH-, -NA¹-, -NCOA¹- oder -NCOOA¹ ersetzt sein können
A¹, A² jeweils unabhängig voneinander Alkyl oder Alkenyl mit 1 bis 10 C-Atomen, das durch 1 bis 5 F- und/oder Cl-Atome substiutiert sein kann, wobei A¹ und A² zusammen auch Cycloalkyl oder Cylcloalkylen mit 3-7 C-Atomen, worin eine CH₂Gruppe durch -O-, -NH-, -NA¹-, NCOA¹- oder-NCOOA¹- ersetzt sein kann,
und
Hal F, Cl, Br oder I
bedeuten,
sowie deren Salze und/oder Solvate.

2. Verbindungen der Formel I gemäß Anspruch 1, **dadurch gekennzeichnet, daß** R¹ und R² unabhängig voneinander H, OA¹ oder Cl, Br oder F bedeuten.

3. Verbindungen der Formel I gemäß Anspruch 1 oder 2, **dadurch gekennzeichnet, daß** R³ und R⁴ unabhängig voneinander H, terminal durch -NH₂, -NHCH₃, -N(CH₃)₂ substituiertes Alkyl mit 1 bis 8 C-Atomen oder eine der folgenden Gruppen: 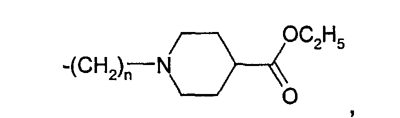 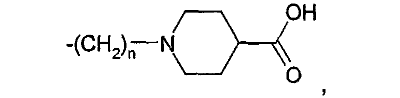 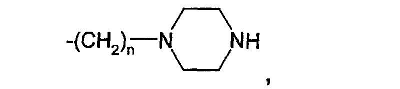 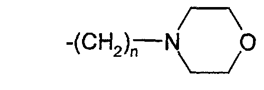 oder 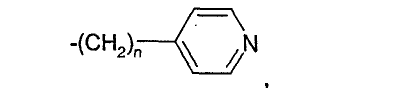 worin n den Wert 0, 1, 2, 3, 4, 5, 6, 7, 8, 9 oder 10 aufweist, bedeutet.

4. Verbindungen der Formel I1 bis 19 gemäß Anspruch 1: 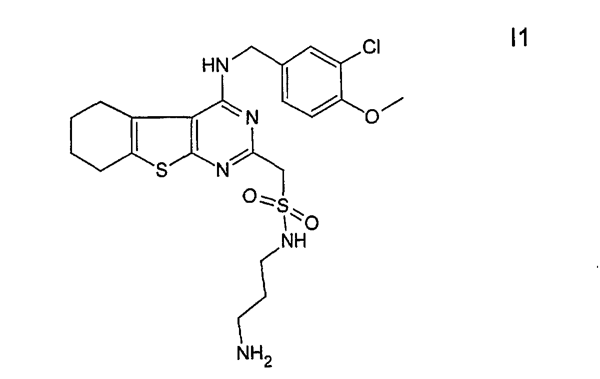 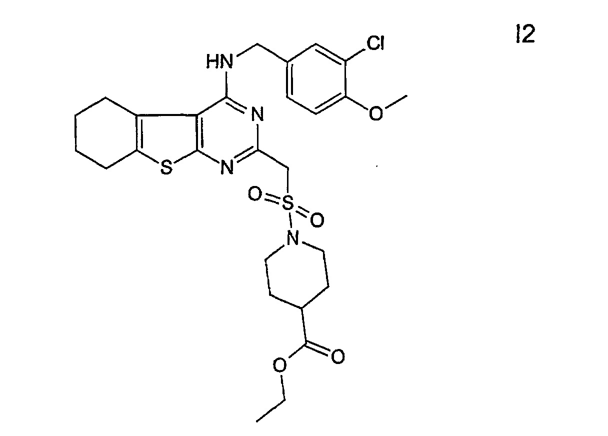 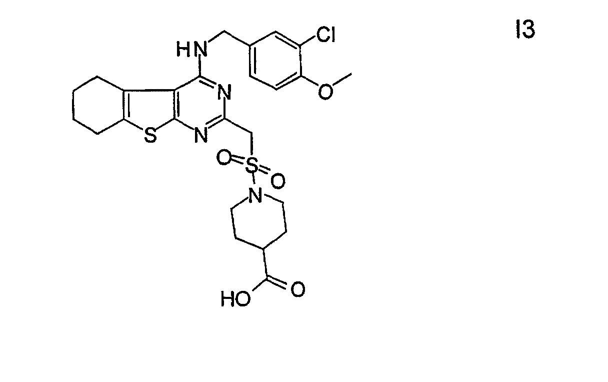 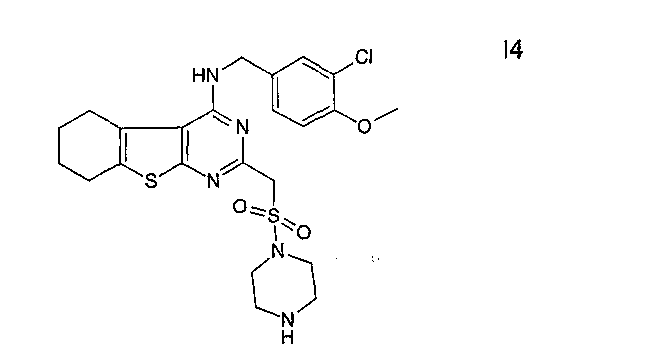 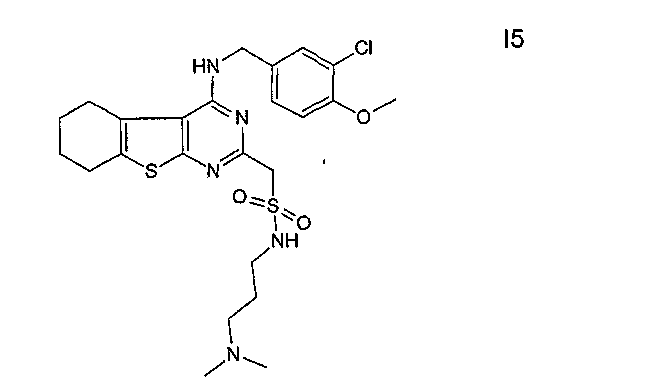 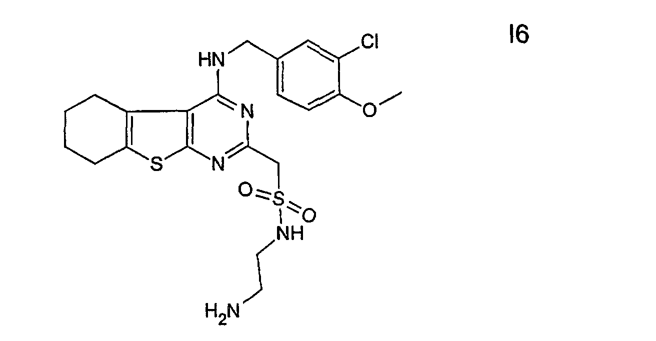 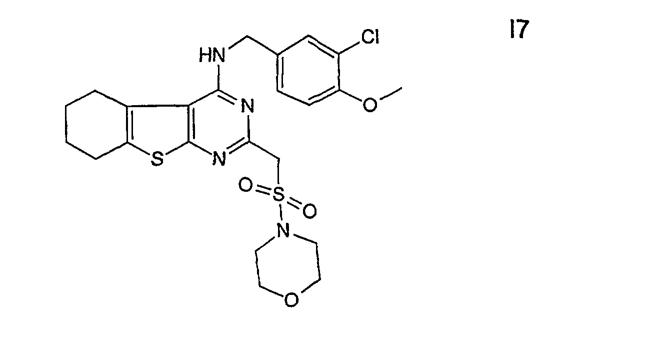 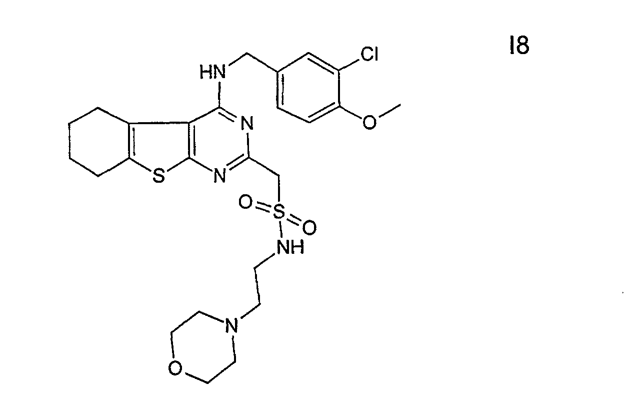 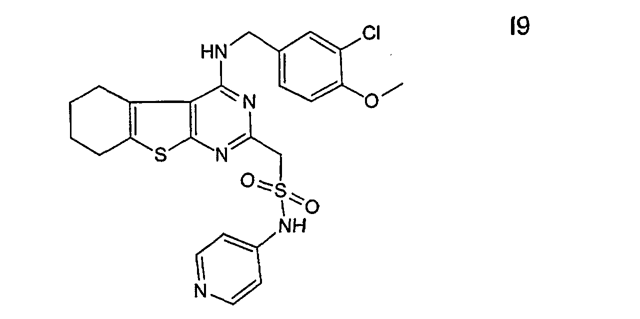 sowie deren Salze und Solvate.

5. Verfahren zur Herstellung von Verbindungen der Formel I nach einem oder mehreren der Ansprüche 1 bis 4, sowie deren Salzen und Solvaten,
**dadurch gekennzeichnet, daß** man
a) eine Verbindung der Formel II 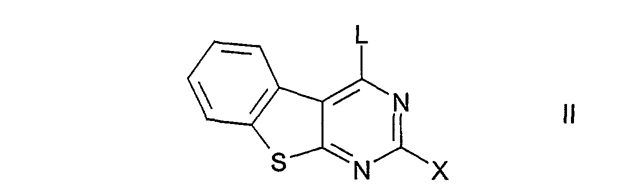 worin
X -CH₂SO₂NR³R⁴,
und
L Cl, Br, OH, SCH₃ oder eine reaktionsfähige veresterte OH-Gruppe bedeutet,
mit einer Verbindung der Formel III 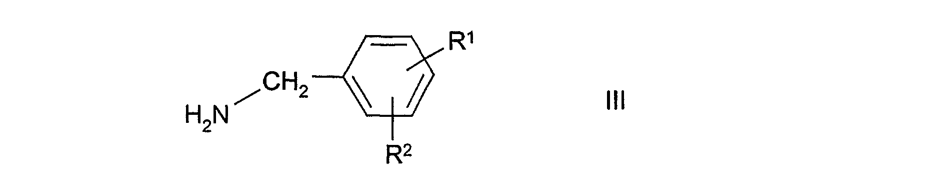 worin
R¹ und R² die angegebenen Bedeutungen haben,
umsetzt,
oder
b) eine Verbindung der Formel IV 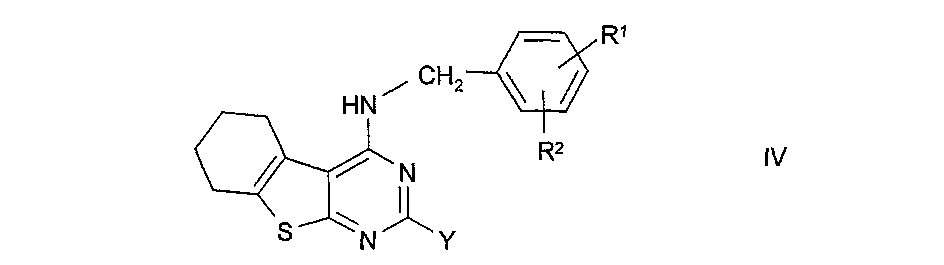 worin
Y -CH₂S0₂Q,
Q Cl, Br, OH, oder eine reaktionsfähige veresterte OH-Gruppe bedeutet
und
R¹ und R² die in Anspruch 1 angegebene Bedeutung aufweisen, mit einer Verbindung der Formel V
HNR³R⁴ V
worin
R³ und R⁴ die in Anspruch 1 angegebenen Bedeutungen haben,
umsetzt,
oder
und/oder daß man eine Verbindung der Formel I in eines ihrer Salze und/oder Solvate überführt.

6. Verbindungen der Formel II 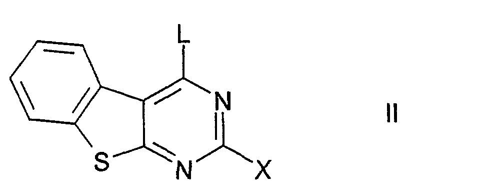 worin
X -CH₂SO₂NR³R⁴,
und
L Cl, Br, OH, SCH₃ oder eine reaktionsfähige veresterte OHGruppe bedeutet.

7. Verbindungen der Formel IV 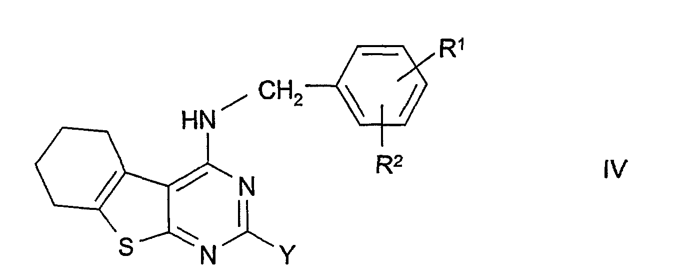 worin
Y -CH₂SO₂Q,
Q Cl, Br, OH, oder eine reaktionsfähige veresterte OH-Gruppe bedeutet
und
R¹ und R² die in Anspruch 1 angegebene Bedeutung aufweisen.

8. Verfahren zur Herstellung pharmazeutischer Zubereitungen, **dadurch gekennzeichnet, daß** man mindestens eine Verbindung der Formel I nach einem oder mehreren der Ansprüche 1 bis 4 und/oder eines ihrer physiologischen unbedenklichen Salze und Solvate zusammen mit mindestens einem festen, flüssigen oder halbflüssigen Träger- oder Hilfsstoff in eine geeignete Dosierungsform bringt.

9. Pharmazeutische Zubereitung, **gekennzeichnet durch** einen Gehalt an mindestens einer Verbindung der Formel I nach einem oder mehreren der Ansprüche 1 bis 4 und/oder einem ihrer physiologisch unbedenklichen Salze und Solvate.

10. Verbindungen der Formel I nach einem oder mehreren der Ansprüche 1 bis 4 und/oder ihre physiologisch unbedenklichen Salze und Solvate zur Behandlung und Prophylaxe von Krankheiten des Herz-Kreislaufsystems und Potenzstörungen.

11. Verbindungen der Formel I nach nach einem oder mehreren der Ansprüche 1 bis 4 und/oder ihre physiologisch unbedenklichen Salze und Solvate als Phosphodiesterase V-Hemmer.

12. Verwendung von Verbindungen der Formel I nach nach einem oder mehreren der Ansprüche 1 bis 4 und/oder ihre physiologisch unbedenklichen Salze und Solvate als Phosphodiesterase V-Hemmer zur Herstellung eines Arzneimittels zur Behandlung oder Prophylaxe von Krankheiten, die durch einen zu geringen cGMP-Spiegel verursacht werden und/oder durch die Erhöhung des cGMP-Spiegels beeinflußt werden können.

13. Verwendung von Verbindungen der Formel I nach einem oder mehreren der Ansprüche 1 bis 4 und/oder ihre physiologisch unbedenklichen Salze und Solvate, zur Herstellung eines Arzneimittels.

14. Verwendung von Verbindungen gemäß einem oder mehreren der Ansprüche 1 bis 4 und/oder ihre physiologisch unbedenklichen Salze und Solvate zur Herstellung eines Arzneimittels zur Behandlung und Prophylaxe von Potentzstörungen, Angina, Bluthochdruck, pulmonalem Hochdruck, congestivem Herzversagen, Herzinfarkt, chronischer obstruktiver pulmonaler Krankheit (COPD), Cor pulmonale, Rechtsherzinsuffizienz, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose, zur Behandlung weiblicher Sexualstörungen, Entzündungen, Osteoporose, zur Behandlung von maligner Hypertonie, Phäochromazytom, peripherer Gefäß(-verschluß)-erkrankungen, Gefäßerkrankungen, Thrombozytopenie, Ulcus pepticum, Darmbewegungsstörungen, perkutaner transluminaler Koronarangioplastie, Karotis Angioplastie, postoperativer Stenose des Koronarbypasses, Vorwehen und benigner Prostata-Hyperplasie.

15. Arzneimittel enthaltend mindestens eine Verbindung der Formel I gemäß einem oder mehreren der Ansprüche 1 bis 4 und/oder ihre physiologisch unbedenklichen Salze und Solvate und mindestens einen weiteren Arzneimitteiwirkstoff.

16. Arzneimittel enthaltend mindestens eine Verbindung der Formel I gemäß einem oder mehreren der Ansprüche 1 bis 4 und/oder ihre physiologisch unbedenklichen Salze und Solvate und mindestens einen weiteren Arzneimittelwirkstoff ausgewählt aus der folgenden Gruppe a) bis k):
a) Prostaglandin oder Prostaglandinderivat,
b) Calcium-Antagonist,
c) Antithromboticum,
d) Endothelin-Rezeptor-Antagonist,
e) Nitrat,
f) α-adrenerger Inhibitor,
g) zentral wirksamer dopaminerger Wirkstoff,
h) ACE-Hemmer,
i) NEP-Hemmer,
j) gemischter α,β-Inhibitor,
k) vasoaktives Intestinalpeptid.

17. Set (Kit), bestehend aus getrennten Packungen von
(a) einer wirksamen Menge an einer Verbindung der Formel I gemäß einem oder mehreren der Ansprüche 1 bis 4 und/oder ihrer physiologisch unbedenklichen Salze und Solvate und
(b) einer wirksamen Menge eines weiteren Arzneimittelswirkstoffs.

18. Verwendung von Verbindungen der Formel I gemäß einem oder mehreren der Ansprüche 1 bis 4 und/oder ihrer physiologisch unbedenklichen Salze und Solvate, zur Herstellung eines Arzneimittels zur Behandlung oder Prophylaxe von Krankheiten des Herz-Kreislaufsystems, zur Behandlung und/oder Prophylaxe von Potenzstörungen, Angina, Bluthochdruck, pulmonalem Hochdruck, congestivem Herzversagen, Herzinfarkt, chronischer obstruktiver pulmonaler Krankheit (COPD), Cor pulmonale, Rechtsherzinsuffizienz, Atherosklerose, Bedingungen verminderter Durchgängigkeit der Herzgefäße, peripheren vaskulären Krankheiten, Schlaganfall, Bronchitis, allergischem Asthma, chronischem Asthma, allergischer Rhinitis, Glaucom, Irritable Bowel Syndrome, Tumoren, Niereninsuffizienz, Leberzirrhose, zur Behandlung weiblicher Sexualstörungen, Entzündungen, Osteoporose, zur Behandlung von maligner Hypertonie, Phäochromazytom, peripherer Gefäß(-verschluß)-erkrankungen, Gefäßerkrankungen, Thrombozytopenie, Ulcus pepticum, Darmbewegungsstörungen, perkutaner transluminaler Koronarangioplastie, Karotis Angioplastie, postoperativer Stenose des Koronarbypasses, Vorwehen und benigner Prostata-Hyperplasie,
in Kombination mit mindestens einem weiteren Arzneimittelwirkstoff.

## Claims

1. Compounds of the formula I 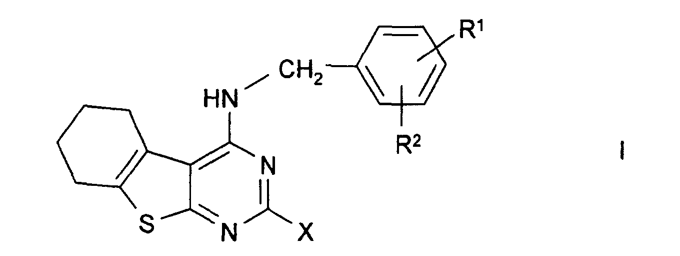 in which
R¹, R² each, independently of one another, denote H, OA¹, OCOA¹, OH or Hal,
where R¹ and R² together also denote alkylene having 3-5 C atoms, -O-CH₂-CH₂-, -CH₂-O-CH₂-, -O-CH₂-O- or -O-CH₂-CH₂-O-,
X denotes -CH₂SO₂NR³R⁴,
R³ R⁴, independently of one another, denote H, a hetero-aromatic radical, alkyl or alkenyl having 1-10 C atoms, each of which is unsubstituted or terminally substituted by -NH₂, NHA¹ or -NA¹A² and in which one or two CH₂ groups may be replaced by -CH=CH- groups, -O-, -NH- or -NA¹-, where R³ and R⁴ together also denote cycloalkyl or cycloalkylene having 3-7 C atoms, each of which is unsubstituted or mono- or polysubstituted by -Hal, -NH₂, -NHA, -NA¹A², -NHCOA¹, NHCOOA¹, -COOH, -COOA¹, -CONH₂, -CONHA¹, or-CONA¹A² and in which one or two -CH₂- groups may be replaced by -O-, -NH-, -NA¹-, -NCOA¹- or -NCOOA¹
A¹, A² each, independently of one another, denote alkyl or alkenyl having 1 to 10 C atoms, each of which may be substituted by 1 to 5 F and/or Cl atoms, where A¹ and A² together also denote cycloalkyl or cycloalkylene having 3-7 C atoms, in which one CH₂ group may be replaced by -O-, -NH-, -NA¹-, NCOA¹- or -NCOOA¹-,
and
Hal denotes F, Cl, Br or I,
and salts and/or solvates thereof.

2. Compounds of the formula I according to Claim 1, **characterised in that** R¹ and R², independently of one another, denote H, OA¹ or Cl, Br or F.

3. Compounds of the formula I according to Claim 1 or 2, **characterised in that** R³ and R⁴, independently of one another, denote H, alkyl having 1 to 8 C atoms which is terminally substituted by -NH₂, -NHCH₃, -N(CH₃)₂, or one of the following groups: 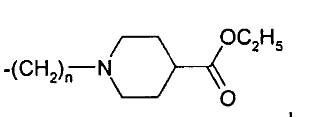 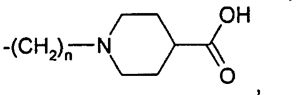 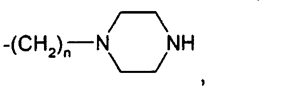 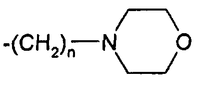 or 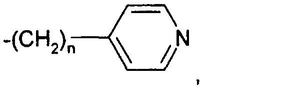 in which n has the value 0, 1, 2, 3, 4, 5, 6, 7, 8, 9 or 10.

4. Compounds of the formulae I1 to 19 according to Claim 1: 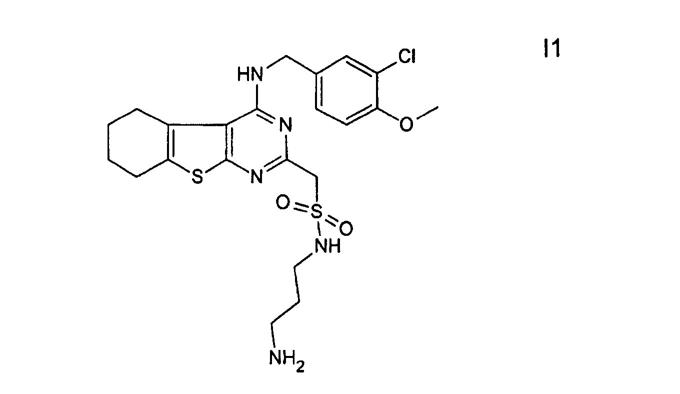 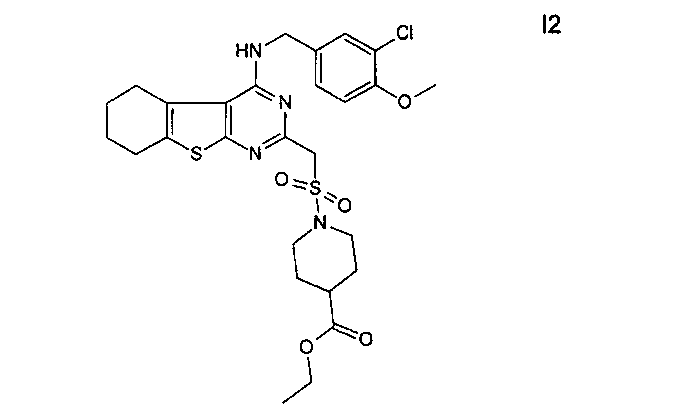 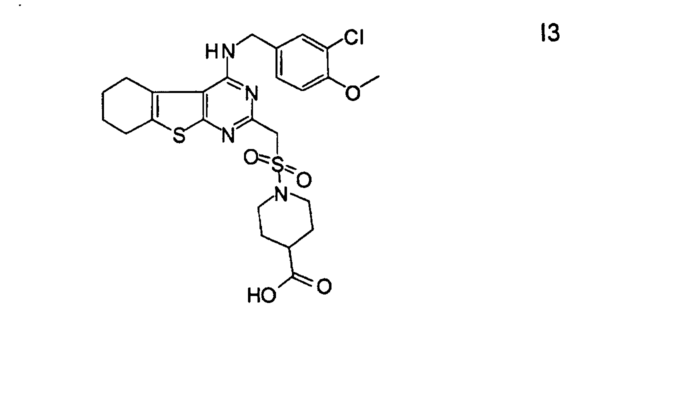 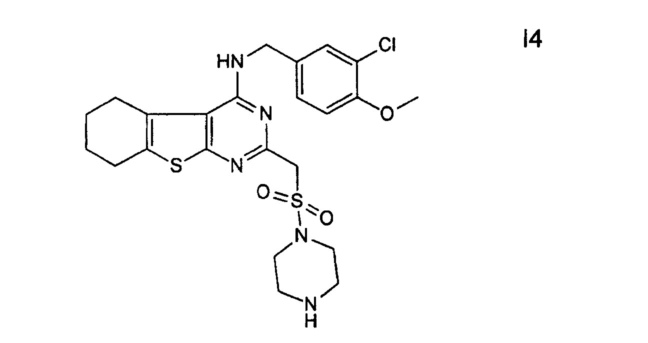 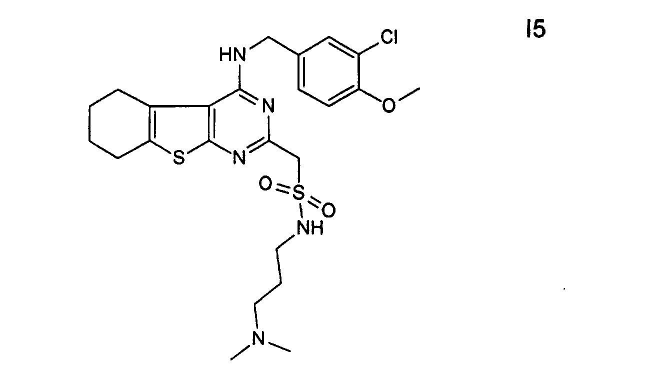 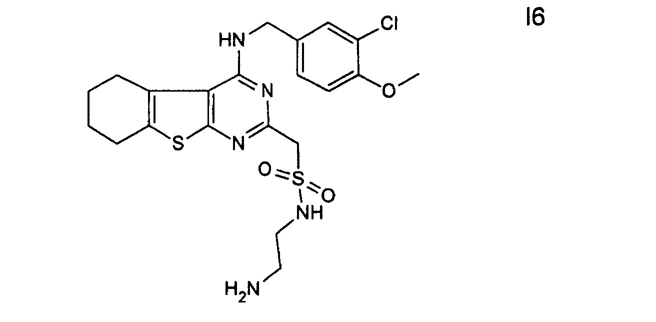 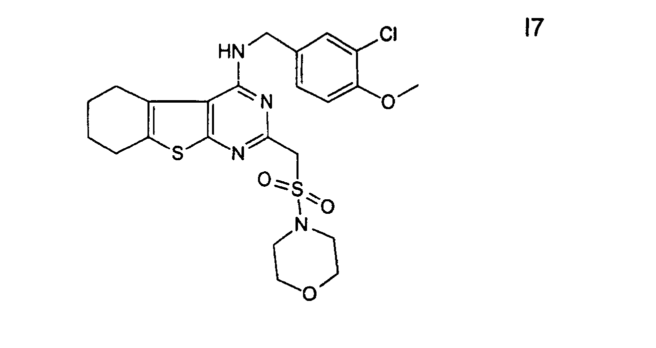 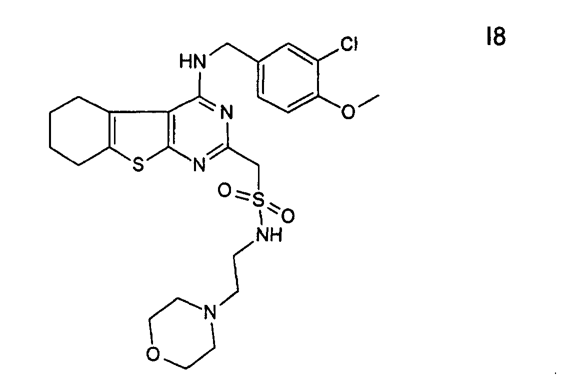 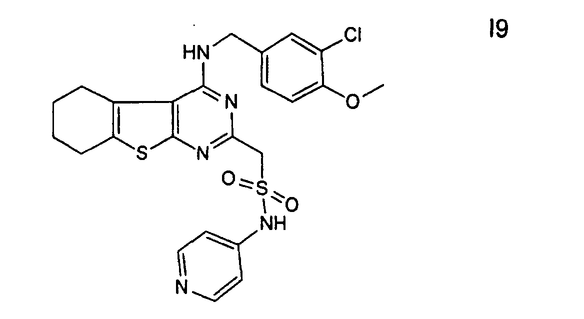 and salts and solvates thereof.

5. Process for the preparation of compounds of the formula I according to one or more of Claims 1 to 4, and salts and solvates thereof,
**characterised in that**
a) a compound of the formula II 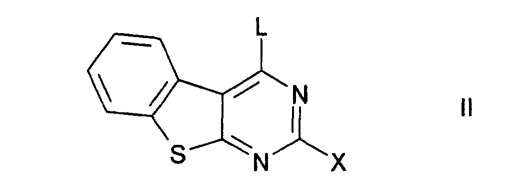 in which
X denotes -CH₂SO₂NR³R⁴,
and
L denotes Cl, Br, OH, SCH₃ or a reactive esterified OH group,
is reacted with a compound of the formula III 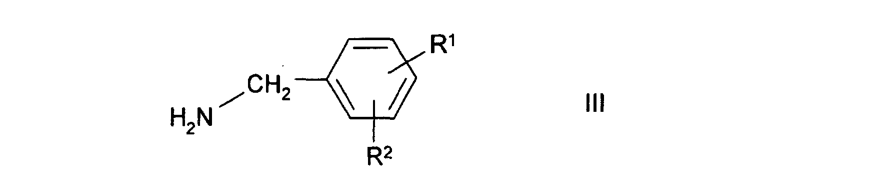 in which
R¹ and R² have the meanings indicated,
or
b) a compound of the formula IV 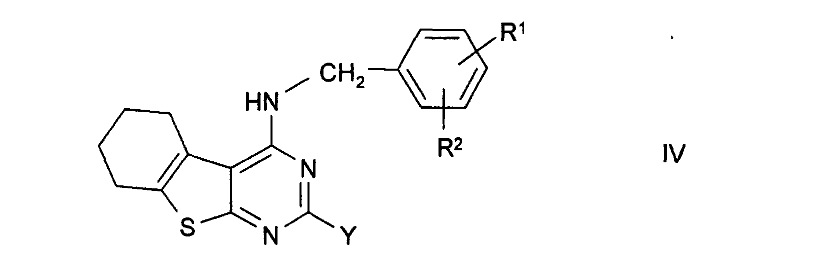
in which
Y denotes -CH₂SO₂Q,
Q denotes Cl, Br, OH, or a reactive esterified OH group
and
R¹ and R² have the meaning indicated in Claim 1,
is reacted with a compound of the formula V
HNR³R⁴ V
in which
R³ and R⁴ have the meanings indicated in Claim 1,
and/or **in that** a compound of the formula I is converted into one of its salts and/or solvates.

6. Compounds of the formula II 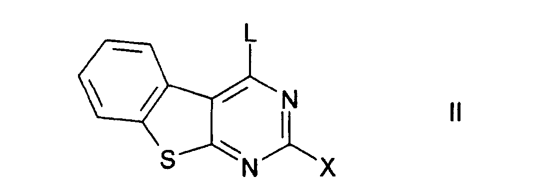 in which
X denotes -CH₂SO₂NR³R⁴,
and
L denotes Cl, Br, OH, SCH₃ or a reactive esterified OH group.

7. Compounds of the formula IV 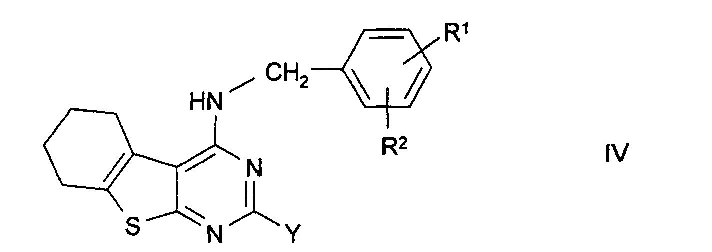 in which
Y denotes -CH₂SO₂Q,
Q denotes Cl, Br, OH, or a reactive esterified OH group
and
R¹ and R² have the meaning indicated in Claim 1.

8. Process for the preparation of pharmaceutical compositions, **characterised in that** at least one compound of the formula I according to one or more of Claims 1 to 4 and/or one of its physiologically acceptable salts and solvates is brought into a suitable dosage form together with at least one solid, liquid or semi-liquid excipient or adjuvant.

9. Pharmaceutical composition, **characterised by** a content of at least one compound of the formula I according to one or more of Claims 1 to 4 and/or one of its physiologically acceptable salts and solvates.

10. Compounds of the formula I according to one or more of Claims 1 to 4 and/or physiologically acceptable salts and solvates thereof for the treatment and prophylaxis of diseases of the cardiovascular system and potency disorders.

11. Compounds of the formula I according to one or more of Claims 1 to 4 and/or physiologically acceptable salts and solvates thereof as phosphodiesterase V inhibitors.

12. Use of compounds of the formula I according to one or more of Claims 1 to 4 and/or physiologically acceptable salts and solvates thereof as phosphodiesterase V inhibitors for the preparation of a medicament for the treatment or prophylaxis of diseases which are caused by an excessively low cGMP level and/or can be influenced by the increase in the cGMP level.

13. Use of compounds of the formula I according to one or more of Claims 1 to 4 and/or physiologically acceptable salts and solvates thereof for the preparation of a medicament.

14. Use of compounds according to one or more of Claims 1 to 4 and/or physiologically acceptable salts and solvates thereof for the preparation of a medicament for the treatment and prophylaxis of potency disorders, angina, high blood pressure, pulmonary hypertension, congestive heart failure, cardiac infarction, chronic obstructive pulmonary disease (COPD), cor pulmonale, dextrocardiac insufficiency, atherosclerosis, conditions of reduced patency of the heart vessels, peripheral vascular diseases, strokes, bronchitis, allergic asthma, chronic asthma, allergic rhinitis, glaucoma, irritable bowel syndrome, tumours, renal insufficiency, liver cirrhosis, for the treatment of female sexual disorders, inflammation, osteoporosis, for the treatment of malignant hypertonia, phaeochromacytoma, peripheral vascular (occlusion) diseases, vascular diseases, thrombocytopenia, ulcus pepticum, peristaltic motion disorders, percutaneous transluminal coronary angioplasty, carotid angioplasty, postoperative stenosis of the coronary bypass, premonitory pains and benign prostate hyperplasia.

15. Medicament comprising at least one compound of the formula I according to one or more of Claims 1 to 4 and/or physiologically acceptable salts and solvates thereof and at least one further medicament active ingredient.

16. Medicament comprising at least one compound of the formula I according to one or more of Claims 1 to 4 and/or physiologically acceptable salts and solvates thereof and at least one further medicament active ingredient selected from the following group a) to k):
a) prostaglandin or prostaglandin derivative,
b) calcium antagonist,
c) antithrombotic,
d) endothelin receptor antagonist,
e) nitrate,
f) α-adrenergic inhibitor,
g) centrally acting dopaminergic active ingredient,
h) ACE inhibitor,
i) NEP inhibitor,
j) mixed α,β-inhibitor,
k) vasoactive intestinal peptide.

17. Set (kit) consisting of separate packs of
(a) an effective amount of a compound of the formula I according to one or more of Claims 1 to 4 and/or physiologically acceptable salts and solvates thereof
and
(b) an effective amount of a further medicament active ingredient.

18. Use of compounds of the formula I according to one or more of Claims 1 to 4 and/or physiologically acceptable salts and solvates thereof for the preparation of a medicament for the treatment or prophylaxis of diseases of the cardiovascular system, for the treatment and/or prophylaxis of potency disorders, angina, high blood pressure, pulmonary hypertension, congestive heart failure, cardiac infarction, chronic obstructive pulmonary disease (COPD), cor pulmonale, dextrocardiac insufficiency, atherosclerosis, conditions of reduced patency of the heart vessels, peripheral vascular diseases, strokes, bronchitis, allergic asthma, chronic asthma, allergic rhinitis, glaucoma, irritable bowel syndrome, tumours, renal insufficiency, liver cirrhosis, for the treatment of female sexual disorders, inflammation, osteoporosis, for the treatment of malignant hypertonia, phaeochromacytoma, peripheral vascular (occlusion) diseases, vascular diseases, thrombocytopenia, ulcus pepticum, peristaltic motion disorders, percutaneous transluminal coronary angioplasty, carotid angioplasty, postoperative stenosis of the coronary bypass, premonitory pains and benign prostate hyperplasia,
in combination with at least one further medicament active ingredient.

## Revendications

1. Composés de la formule I 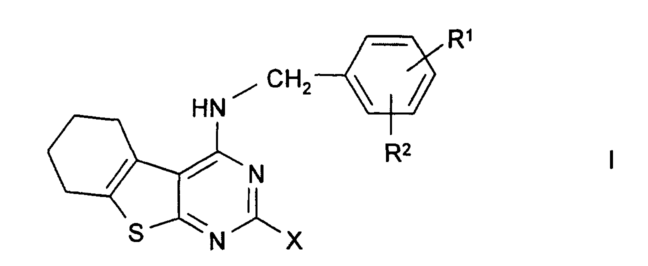 dans laquelle
R¹, R² représentent, chacun indépendamment l'un de l'autre, H, OA¹, OCOA¹, OH ou Hal,
où R¹ et R² ensemble représentent également alkylène comportant de 3 à 5 atomes de C, -O-CH₂-CH₂-, -CH₂-O-CH₂-, -O-CH₂-O- ou -O-CH₂-CH₂-O-,
X représente -CH₂SO₂NR³R⁴,
R³, R⁴, indépendamment l'un de l'autre, représentent H, un radical hétéroaromatique, alkyle ou alkényle comportant de 1 à 10 atomes de C, dont chacun est non substitué ou substitué de façon terminale par -NH₂, NHA¹ ou -NA¹A² et où un ou deux groupes CH₂ peuvent être remplacés par des groupes -CH=CH-, -O-, -NH- ou -NA¹-, où R³ et R⁴ ensemble représentent également cycloalkyle ou cycloalkylène comportant de 3 à 7 atomes de C, dont chacun est non substitué ou mono- ou polysubstitué par -Hal, -NH₂, -NHA, -NA¹A², -NHCOA¹, NHCOOA¹, -COOH, -COOA¹, -CONH₂, -CONHA¹, ou -CONA¹A² et où un ou deux groupes -CH₂- peuvent être remplacés par -O-, -NH-, -NA¹-, -NCOA¹- ou -NCOOA¹
A¹, A² représentent, chacun indépendamment l'un de l'autre, alkyle ou alkényle comportant de 1 à 10 atomes de C, dont chacun peut être substitué par 1 à 5 atomes de F et/ou de Cl, où A¹ et A² ensemble représentent également cycloalkyle ou cycloalkylène comportant de 3 à 7 atomes de C, où un groupe CH₂ peut être remplacé par -O-, -NH-, -NA¹-, NCOA¹- ou -NCOOA¹-,
et
Hal représente F, Cl, Br ou I,
et leurs sels et/ou solvates.

2. Composés de la formule I selon la revendication 1, **caractérisés en ce que** R¹ et R², indépendamment l'un de l'autre, représentent H, OA¹ ou Cl, Br ou F.

3. Composés de la formule I selon la revendication 1 ou 2, **caractérisés en ce que** R³ et R⁴, indépendamment l'un de l'autre, représentent H, alkyle comportant de 1 à 8 atomes de C qui est substitué de façon terminale par -NH₂, -NHCH₃, -N(CH₃)₂, ou un des groupes qui suivent: 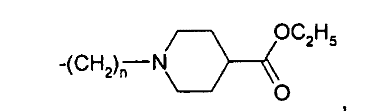 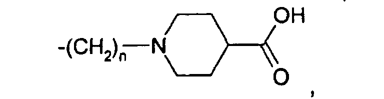 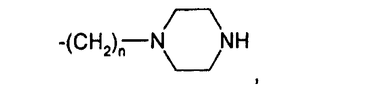 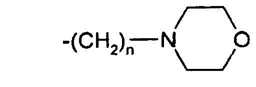 or 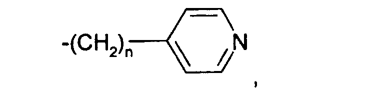 où n présente la valeur 0, 1, 2, 3, 4, 5, 6, 7, 8, 9 ou 10.

4. Composés des formules I1 à 19 selon la revendication 1: 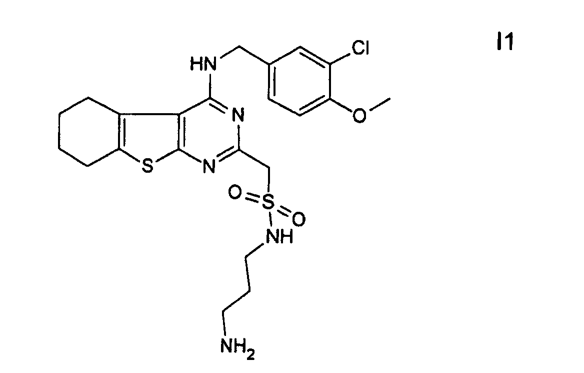 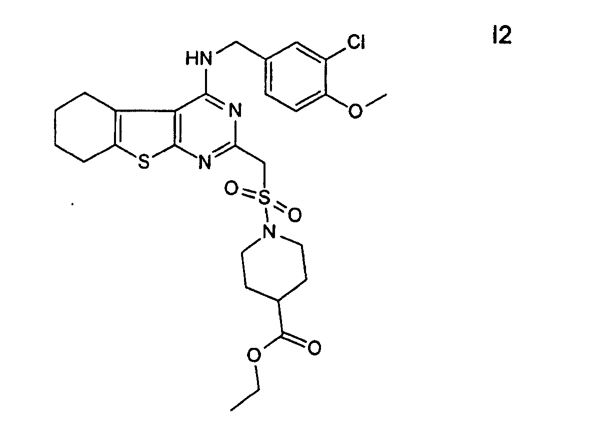 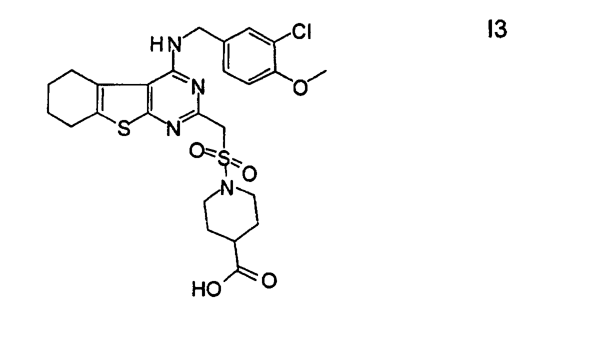 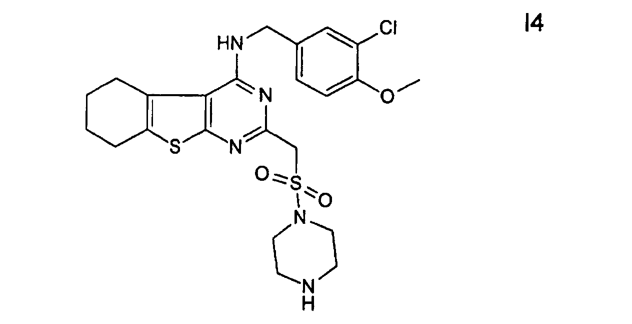 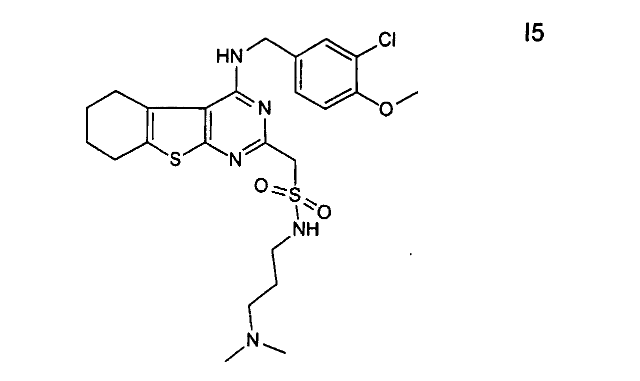 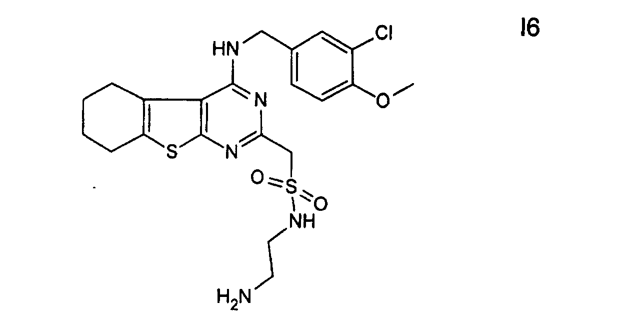 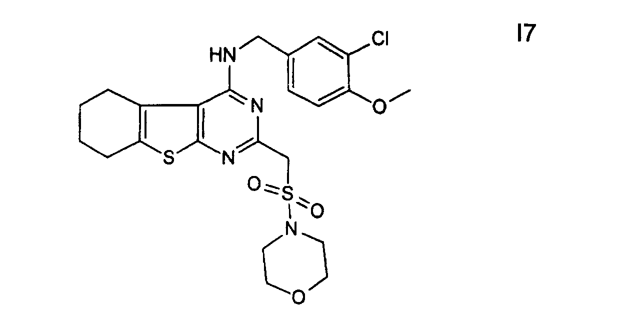 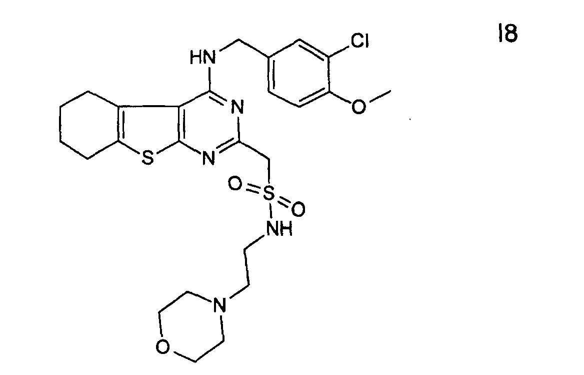 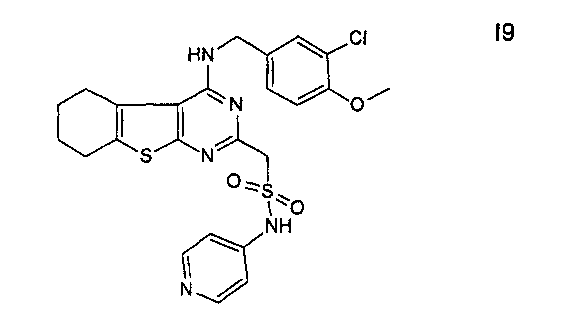 et leurs sels et solvates.

5. Procédé pour la préparation de composés de la formule I selon une ou plusieurs des revendications 1 à 4, et de leurs sels et solvates,
**caractérisé en ce que**
a) un composé de la formule II 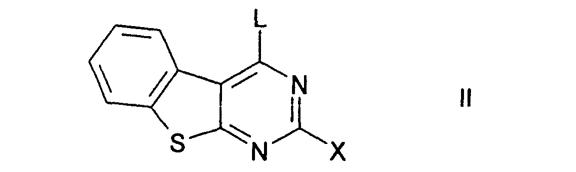 dans laquelle
X représente -CH₂SO₂NR³R⁴,
et
L représente Cl, Br, OH, SCH₃ ou un groupe OH estérifié réactif,
est amené à réagir avec un composé de la formule III 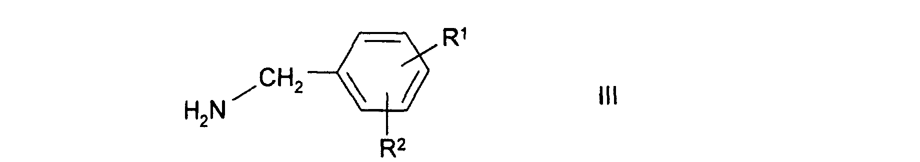 dans laquelle
R¹ et R² présentent les significations indiquées,
ou
b) un composé de la formule IV 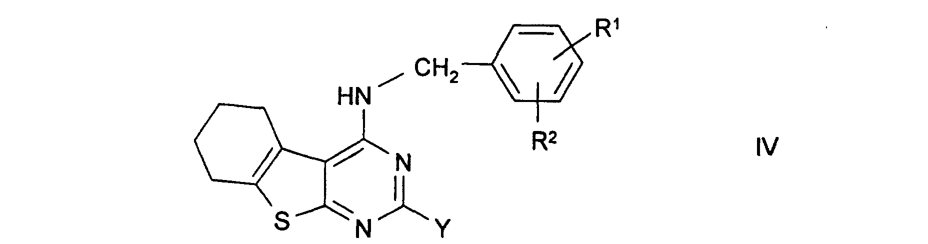
dans laquelle
Y représente -CH₂SO₂Q,
Q représente Cl, Br, OH, ou un groupe OH estérifié réactif
et
R¹ et R² présentent les significations indiquées dans la revendication 1,
est amené à réagir avec un composé de la formule V
HNR³R⁴ V
dans laquelle
R³ et R⁴ présentent les significations indiquées dans la revendication 1,
et/ou **en ce qu'**un composé de la formule I est converti selon l'un de ses sels et/ou solvates.

6. Composés de la formule II 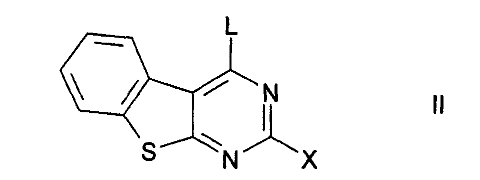 dans laquelle
X représente -CH₂SO₂NR³R⁴,
et
L représente Cl, Br, OH, SCH₃ ou un groupe OH estérifié réactif.

7. Composés de la formule IV 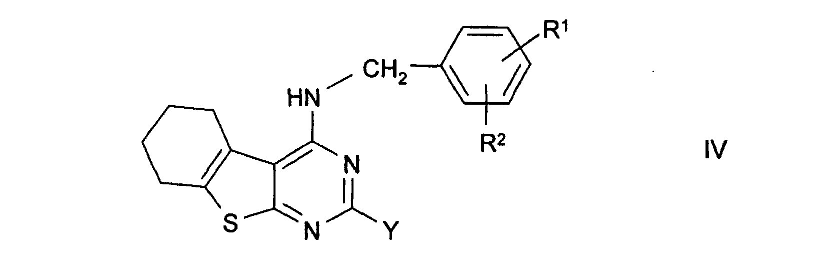 dans laquelle
Y représente -CH₂SO₂Q,
Q représente Cl, Br, OH, ou un groupe OH estérifié réactif
et
R¹ et R² présentent les significations indiquées dans la revendication 1.

8. Procédé pour la préparation de compositions pharmaceutiques, **caractérisé en ce qu'**au moins un composé de la formule I selon une ou plusieurs des revendications 1 à 4 et/ou un de ses sels et solvates physiologiquement acceptables sont amenés selon une forme de dosage appropriée en association avec au moins un excipient ou adjuvant solide, liquide ou semi-liquide.

9. Composition pharmaceutique, **caractérisée par** une teneur en au moins un composé de la formule I selon une ou plusieurs des revendications 1 à 4 et/ou en un de ses sels et solvates physiologiquement acceptables.

10. Composés de la formule I selon une ou plusieurs des revendications 1 à 4 et/ou leurs sels et solvates physiologiquement acceptables pour le traitement et la prophylaxie de maladies du système cardio-vasculaire et de troubles de la puissance sexuelle.

11. Composés de la formule I selon une ou plusieurs des revendications 1 à 4 et/ou leurs sels et solvates physiologiquement acceptables en tant qu'inhibiteurs de phosphodiestérase V.

12. Utilisation de composés de la formule I selon une ou plusieurs des revendications 1 à 4 et/ou de leurs sels et solvates physiologiquement acceptables en tant qu'inhibiteurs de phosphodiestérase V pour la préparation d'un médicament pour le traitement ou la prophylaxie de maladies qui sont générées par un niveau excessivement bas de cGMP et/ou qui peuvent être influencées par l'augmentation du niveau de cGMP.

13. Utilisation de composés de la formule I selon une ou plusieurs des revendications 1 à 4 et/ou de leurs sels et solvates physiologiquement acceptables pour la préparation d'un médicament.

14. Utilisation de composés de la formule I selon une ou plusieurs des revendications 1 à 4 et/ou de leurs sels et solvates physiologiquement acceptables pour la préparation d'un médicament pour le traitement et la prophylaxie de troubles de la puissance sexuelle, de l'angine de poitrine, de l'hypertension artérielle, de l'hypertension pulmonaire, de la défaillance du coeur par congestion, de l'infarctus du myocarde, de la bronchopneumopathie chronique obstructive (COPD), de troubles du coeur pulmonaire, de l'insuffisance dextro-cardiaque, de l'athérosclérose, des conditions de passage réduit des vaisseaux du coeur, des maladies vasculaires périphériques, des accidents vasculaires cérébraux, de la bronchite, de l'asthme allergique, de l'asthme chronique, de la rhinite allergique, du glaucome, du syndrome du colon irritable, des tumeurs, de l'insuffisance rénale, de la cirrhose du foie, pour le traitement des troubles sexuelles de la femme, de l'inflammation, de l'ostéoporose, pour le traitement de l'hypertonie maligne, de la phaeochromacytoma, des maladies vasculaires périphériques (occlusion), des maladies vasculaires, de la thrombocytopénie, de l'ulcus pepticum, des troubles du mouvement péristaltique, de l'angioplastie coronarienne percutanée trans-luminale, de l'angioplastie de la carotide, de la sténose postopératoire du pontage coronarien, des algies prémonitoires et de l'hyperplasie bénigne de la prostate.

15. Médicament comprenant au moins un composé de la formule I selon une ou plusieurs des revendications 1 à 4 et/ou leurs sels et solvates physiologiquement acceptables et au moins un autre ingrédient actif de médicament.

16. Médicament comprenant au moins un composé de la formule I selon une ou plusieurs des revendications 1 à 4 et/ou leurs sels et solvates physiologiquement acceptables et au moins un autre ingrédient actif de médicament choisi parmi le groupe qui suit a) à k):
a) prostaglandine ou dérivé de prostaglandine,
b) antagoniste du calcium,
c) antithrombotique,
d) antagoniste de récepteur d'endothéline,
e) nitrate,
f) inhibiteur α-adrénergique,
g) ingrédient actif dopaminergique agissant centralement,
h) inhibiteur ACE,
i) inhibiteur NEP,
j) inhibiteur α,β mélangé,
k) peptide intestinal vasoactif.

17. Ensemble (kit) constitué par des conditionnements séparés de
(a) une quantité efficace d'un composé de la formule I selon une ou plusieurs des revendications 1 à 4 et/ou de leurs sels et solvates physiologiquement acceptables
et
(b) une quantité efficace d'un autre ingrédient de médicament actif.

18. Utilisation de composés de la formule I selon une ou plusieurs des revendications 1 à 4 et/ou de leurs sels et solvates physiologiquement acceptables pour la préparation d'un médicament pour le traitement et la prophylaxie de troubles de la puissance sexuelle, de l'angine de poitrine, de l'hypertension artérielle, de l'hypertension pulmonaire, de la défaillance du coeur par congestion, de l'infarctus du myocarde, de la bronchopneumopathie chronique obstructive (COPD), de troubles du coeur pulmonaire, de l'insuffisance dextro-cardiaque, de l'athérosclérose, des conditions de passage réduit des vaisseaux du coeur, des maladies vasculaires périphériques, des accidents vasculaires cérébraux, de la bronchite, de l'asthme allergique, de l'asthme chronique, de la rhinite allergique, du glaucome, du syndrome du colon irritable, des tumeurs, de l'insuffisance rénale, de la cirrhose du foie, pour le traitement des troubles sexuelles de la femme, de l'inflammation, de l'ostéoporose, pour le traitement de l'hypertonie maligne, de la phaeochromacytoma, des maladies vasculaires périphériques (occlusion), des maladies vasculaires, de la thrombocytopénie, de l'ulcus pepticum, des troubles du mouvement péristaltique, de l'angioplastie coronarienne percutanée transluminale, de l'angioplastie de la carotide, de la sténose postopératoire du pontage coronarien, des algies prémonitoires et de l'hyperplasie bénigne de la prostate,
en combinaison avec au moins un autre ingrédient de médicament actif.
